(19) Europäisches Patentamt
European Patent Office
Office européen des brevets

(11) Publication number: 0 302 626 A2

# (12) EUROPEAN PATENT APPLICATION

(21) Application number: 88306643.3

(22) Date of filing: 20.07.88

(51) Int. Cl.[4]: C07C 69/74 , C07C 69/743 , C07C 121/66 , C07C 117/00 , C07C 149/273 , C07C 109/14

(30) Priority: 06.08.87 GB 8718621
03.06.88 GB 8813210

(43) Date of publication of application: 08.02.89 Bulletin 89/06

(84) Designated Contracting States: AT BE CH DE ES FR GB GR IT LI LU NL SE

(71) Applicant: IMPERIAL CHEMICAL INDUSTRIES PLC
Imperial Chemical House Millbank
London SW1P 3JF(GB)

(72) Inventor: Robson, Michael John
12 Greenham Wood North Lake
Bracknell Berkshire RG12 4WJ(GB)
Inventor: Spinney, Mark Andrew
3 Sheriden Way Woosehill
Wokingham Berkshire(GB)

(74) Representative: Bishop, Nigel Douglas et al
Imperial Chemical Industries PLC Legal Department: Patents PO Box 6 Bessemer Road
Welwyn Garden City Herts, AL7 1HD(GB)

## (54) Insecticidal compounds.

(57) The invention provides novel, insecticidally active compounds of formula (I) :

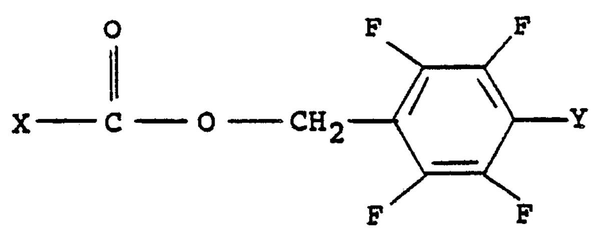

(I)

wherein X represents a group of formula :

EP 0 302 626 A2

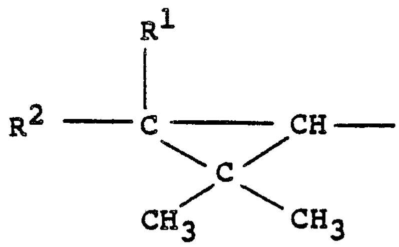

where either (i) $R^1$ and $R^2$ represent hydrogen, methyl, halo or haloalkyl, or (ii) $R^1$ represents hydrogen and $R^2$ represents either a group of formula $R^3(R^4)C=CH-$ or a group of formula $R^3(R^4)(R^5)C-CH(R^5)-$, wherein $R^3$ and $R^4$ represent hydrogen, methyl, halo or haloalkyl and $R^5$ represent chloro or bromo; and Y represents $-C\equiv CR^{10}$ or $-NH-N=C(R^{11})R^{12}$ where $R^{10}$, $R^{11}$ and $R^{12}$ are selected from hydrogen, alkyl and haloalkyl, or Y represents $-CH_2Z$ where Z is selected from halo, CN, $N_3$, $CH_2CN$, $-OCOR^6$, $-SCOR^7$, $-OCO_2R^7$, $-OR^8$, $-SR^8$, $-N(R^{15})R^8$, $-OSO_2R^9$ and $-ON=C(R^{13})R^{14}$, where $R^6$ represents H, alkyl or haloalkyl, $R^7$ and $R^9$ represent alkyl or haloalkyl, $R^8$ represents propargyl optionally substituted with one or more methyl groups, and $R^{13}$, $R^{14}$ and $R^{15}$ represent alkyl.

The invention also provides insecticidal compositions comprising the compounds of formula (I) and methods of controlling insect pests therewith, and processes and intermediates for their preparation.

# INSECTICIDAL COMPOUNDS

This invention relates to novel fluorobenzyl esters useful in combating insects and similar invertebrate pests, to processes and intermediates for their preparation, to compositions comprising them and to methods of combating insect and similar invertebrate pests using them.

The present invention relates to certain novel fluorobenzyl esters of cyclopropanecarboxylic acids which show a high level of contact, residual and fumigant, insecticidal activity. Many of the compounds have also demonstrated a high level of knockdown activity against a number of public health insect pests.

In a first aspect, the invention provides a compound having the general formula (I) :

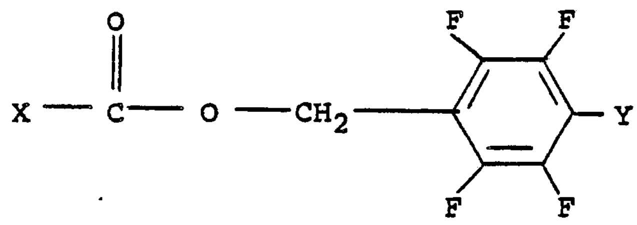

(I)

or a stereoisomer thereof, wherein X represents a group of formula :

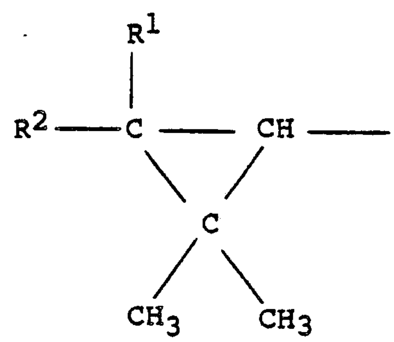

Where either (i) $R^1$ and $R^2$ are each selected from hydrogen, halo and $C_{1-4}$ alkyl or (ii) $R^1$ is hydrogen and $R^2$ represents either a group of formula :

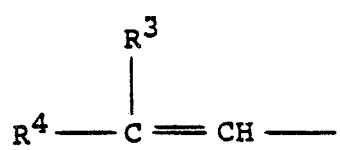

or a group of formula :

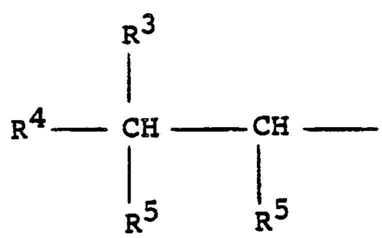

where $R^3$ and $R^4$ are each selected from hydrogen, methyl, halo and $C_{1-2}$ haloalkyl containing at least two fluorine atoms, and $R^5$ is selected from chloro and bromo, and either

(i) Y represents a group of formula $-CH_2-Z$ wherein Z is selected from halo, cyano, azido and cyanomethyl, or Z is selected from a group of formula $-OCOR^6$, a group of formula $-SCOR^7$, a group of formula $-OCO_2R^7$, a group of formula $-OR^8$, a group of formula $-SR^8$, a group of formula $-N(R^{15})R^8$, a group of formula $-OSO_2R^9$, and a group of formula $-ON=C(R^{13})R^{14}$, wherein $R^6$ represents hydrogen, $C_{1-4}$ alkyl or $C_{1-4}$ haloalkyl, $R^8$ represents propargyl optionally substituted with one or more methyl groups, $R^7$ and $R^9$ represent $C_{1-4}$ alkyl or $C_{1-4}$ haloalkyl, and $R^{13}$, $R^{14}$ and $R^{15}$ represent $C_{1-4}$ alkyl, or

(ii) Y represents a group of formula $-C{\equiv}CR^{10}$ or a group of formula $-NH-N=C(R^{11})R^{12}$ wherein $R^{10}$, $R^{11}$ and $R^{12}$ are selected from hydrogen, $C_{1-4}$ alkyl and $C_{1-4}$ haloalkyl.

The compounds according to formula (I) may exist in a number of stereoisomeric forms resulting from the nature of the group X. Thus, except where $R_1$ and $R_2$ are identical, the carbon atoms at the 1-position of the cyclopropane ring may be asymmetrically substituted and may exist in the R-configuration or the S-configuration. Similarly the carbon atom at the 3-position of the cyclopropane ring may also exist in the R-configuration or the S-configuration when the groups $R^1$ and $R^2$ are not identical; the presence of two such asymmetrically substituted centres in the cyclopropane ring is additionally characterised by the existence of cis and trans isomers, according to the relative configuration of the ring substituents. Where the group $R^2$ contains a carbon-carbon double bond, there exists the further possibility of E- and Z-isomeric forms.

Those skilled in the art will recognise that different isomeric forms of the compounds according to formula (I) exhibit different biological characteristics, for example differing levels of insecticidal activity. All individual isomeric forms and mixtures thereof, including racemates, arising from stereoisomerism in the group X or elsewhere in the structure of compounds according to formula (I) are within the scope of the invention.

Preferred compounds according to the invention are those wherein X represents the group

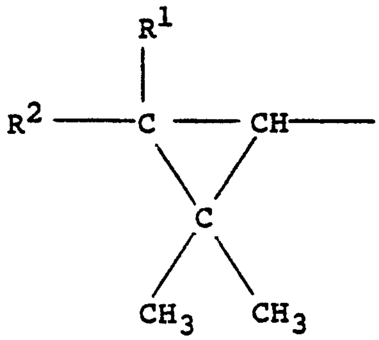

in which ether (i) $R^1$ and $R^2$ both represent methyl, or (ii) $R^1$ represents hydrogen and $R^2$ represents the group of formula $R^4(R^3)C=CH-$ in which $R^3$ and $R^4$ are each selected from hydrogen, methyl, halo and trifluoromethyl.

Examples of esters according to the invention are those derived from the following acids, preferably in their racemic or (1R) forms in those cases where the groups $R^1$ and $R^2$ in formula (I) are not equivalent :

(trans)-3-(Z-2-chloro-3,3,3-trifluoroprop-1-en-1-yl)-2,2-dimethylcyclopropanecarboxylic acid,
(cis)-3-(Z-2-chloro-3,3,3-trifluoroprop-1-en-1-yl)-2,2-dimethylcyclopropanecarboxylic acid,
(trans)-3-(2,2-dichloroethenyl)-2,2-dimethylcyclopropanecarboxylic acid,
(cis)-3-(2,2-dichloroethenyl)-2,2-dimethylcyclopropanecarboxylic acid,
(trans)-3-(Z-2-bromo-3,3,3-trifluoroprop-1-en-1-yl)-2,2-dimethylcyclopropanecarboxylic acid,
(cis)-3-(Z-2-bromo-3,3,3-trifluoroprop-1-en-1-yl)-2,2-dimethylcyclopropanecarboxylic acid,
(trans)-3-(Z-2,3,3,3-tetrafluoroprop-1-en-1-yl)-2,2-dimethylcyclopropanecarboxylic acid,
(cis)-3-(Z-2,3,3,3-tetrafluoroprop-1-en-1-yl)-2,2-dimethylcyclopropanecarboxylic acid,
2,2,3,3-tetramethylcyclopropanecarboxylic acid,
(trans)-3-(2,2-dibromoethenyl)-2,2-dimethylcyclopropanecarboxylic acid,
(cis)-3-(2,2-dibromoethenyl)-2,2-dimethylcyclopropanecarboxylic acid,
(trans)-3-(2,2-difluoroethenyl)-2,2-dimethylcyclopropanecarboxylic acid,
(cis)-3-(2,2-difluoroethenyl)-2,2-dimethylcyclopropane carboxylic acid.
(trans)-3-(2-fluoroethenyl)-2,2-dimethylcyclopropanecarboxylic acid,
(cis)-3-(2-fluoroethenyl)-2,2-dimethylcyclopropanecarboxylic acid,

(trans)-3-(2-methylprop-1-en-1-yl)-2,2-dimethylcyclopropanecarboxylic acid, and
(cis)-3-(2-methylprop-1-en-1-yl)-2,2-dimethylcyclopropanecarboxylic acid.

Particularly preferred compounds according to the invention are those of formula (I) wherein the group Y has a value selected from fluoromethyl, chloromethyl, bromomethyl, cyanomethyl, azidomethyl, 2-cyanoethyl, formyloxymethyl, acetoxymethyl, acetylthiomethyl, trifluoroacetoxymethyl, methoxycarbonyloxymethyl, (1-methylprop-2-yn-1-yl)oxymethyl, (but-2-yn-1-yl)oxymethyl, (prop-2-yn-1-yl)oxymethyl, (prop-2-yn-1-yl)thiomethyl, [N-(prop-2-yn-1-yl)-N-methyl]aminomethyl, methanesulphonyloxymethyl, (prop-2-ylideneamino)oxymethyl, ethynyl, prop-2-ylidenehydrazino and ethylidenehydrazino.

Particular compounds according to formula (I) include those set out, by way of example only, in Table I, in their (1R,cis), (1R,trans). (1RS,cis) and (1RS,trans) stereoisomeric forms where appropriate.

TABLE I

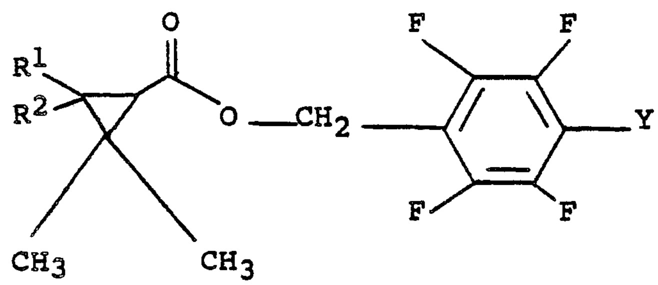

| COMPOUND NO. | $R^1$ | $R^2$ | Y |
|---|---|---|---|
| 1 | $CF_3(Cl)C{=}CH$ | H | $CH_2F$ |
| 2 | $CF_3(Cl)C{=}CH$ | H | $CH_2Cl$ |
| 3 | $CF_3(Cl)C{=}CH$ | H | $CH_2Br$ |
| 4 | $CF_3(Cl)C{=}CH$ | H | $CH_2CN$ |
| 5 | $CF_3(Cl)C{=}CH$ | H | $CH_2N_3$ |
| 6 | $CF_3(Cl)C{=}CH$ | H | $CH_2OCHO$ |
| 7 | $CF_3(Cl)C{=}CH$ | H | $CH_2OCOCH_3$ |
| 8 | $CF_3(Cl)C{=}CH$ | H | $CH_2OCOCF_3$ |
| 9 | $CF_3(Cl)C{=}CH$ | H | $CH_2OCOOCH_3$ |
| 10 | $CF_3(Cl)C{=}CH$ | H | $CH_2OSO_2CH_3$ |
| 11 | $CF_3(Cl)C{=}CH$ | H | $C{\equiv}CH$ |
| 12 | $CF_3(Cl)C{=}CH$ | H | $NHN{=}CHCH_3$ |
| 13 | $CF_3(Cl)C{=}CH$ | H | $NHN{=}C(CH_3)_2$ |
| 14 | $CCl_2{=}CH$ | H | $NHN{=}C(CH_3)_2$ |
| 15 | $CCl_2{=}CH$ | H | $C{\equiv}CH$ |
| 16 | $CCl_2{=}CH$ | H | $CH_2OSO_2CH_3$ |
| 17 | $CCl_2{=}CH$ | H | $CH_2OCOCH_3$ |

TABLE I (Cont/d)

| COMPOUND NO. | $R^1$ | $R^2$ | Y |
|---|---|---|---|
| 18 | $CCl_2=CH$ | H | $CH_2CN$ |
| 19 | $CCl_2=CH$ | H | $CH_2Br$ |
| 20 | $CCl_2=CH$ | H | $CH_2Cl$ |
| 21 | $CCl_2=CH$ | H | $CH_2F$ |
| 22 | $CF_3(Br)C=CH$ | H | $C\equiv CH$ |
| 23 | $CF_3(Br)C=CH$ | H | $NHN=CHCH_3$ |
| 24 | $CF_3(Br)C=CH$ | H | $CH_2OSO_2CH_3$ |
| 25 | $CF_3(Br)C=CH$ | H | $CH_2OCOCH_3$ |
| 26 | $CF_3(Br)C=CH$ | H | $CH_2Br$ |
| 27 | $CF_3(Br)C=CH$ | H | $CH_2Cl$ |
| 28 | $CF_3(Br)C=CH$ | H | $CH_2F$ |
| 29 | $CF_3(Br)C=CH$ | H | $CH_2CN$ |
| 30 | $CF_3(F)C=CH$ | H | $CH_2Cl$ |
| 31 | $CF_3(F)C=CH$ | H | $CH_2F$ |
| 32 | $CF_3(F)C=CH$ | H | $CH_2Br$ |
| 33 | $CF_3(F)C=CH$ | H | $CH_2CN$ |
| 34 | $CF_3(F)C=CH$ | H | $CH_2OCOCH_3$ |
| 35 | $CF_3(F)C=CH$ | H | $NHN=CHCH_3$ |
| 36 | $CF_3(F)C=CH$ | H | $C\equiv CH$ |
| 37 | $CH_3$ | $CH_3$ | $C\equiv CH$ |
| 38 | $CH_3$ | $CH_3$ | $CH_2F$ |
| 39 | $CH_3$ | $CH_3$ | $CH_2Cl$ |
| 40 | $CH_3$ | $CH_3$ | $CH_2Br$ |
| 41 | $CF_3(Cl)C=CH$ | H | $CH_2OCH_2C\equiv CH$ |
| 42 | $CF_3(Cl)C=CH$ | H | $CH_2OCH_2C\equiv CCH_3$ |

TABLE I (Cont/d)

| COMPOUND NO. | $R^1$ | $R^2$ | Y |
|---|---|---|---|
| 43 | $CF_3(Cl)C=CH$ | H | $CH_2SCH_2C\equiv CH$ |
| 44 | $CF_3(Cl)C=CH$ | H | $CH_2ON=C(CH_3)_2$ |
| 45 | $CF_3(Cl)C=CH$ | H | $CH_2N(CH_3)CH_2C\equiv CH$ |
| 46 | $CF_3(Cl)C=CH$ | H | $CH_2-SCOCH_3$ |
| 47 | $CF_3(Cl)C=CH$ | H | $CH_2CH_2CN$ |
| 48 | $CCl_2=CH$ | H | $CH_2SCH_2C\equiv CH$ |
| 49 | $CF_3(F)C=CH$ | H | $CH_2N_3$ |
| 50 | $CFH=CH$ | H | $C\equiv CH$ |
| 51 | $CFH=CH$ | H | $CH_2F$ |
| 52 | $CFH=CH$ | H | $CH_2OCOCH_3$ |
| 53 | $(CH_3)_2C=CH$ | H | $CH_2F$ |
| 54 | $(CH_3)_2C=CH$ | H | $C\equiv CH$ |
| 55 | $(CH_3)_2C=CH$ | H | $CH_2OCOCH_3$ |
| 56 | $CBr_2=CH$ | H | $C\equiv CH$ |
| 57 | $CBr_2=CH$ | H | $CH_2F$ |
| 58 | $CBr_2=CH$ | H | $CH_2OCOCH_3$ |
| 59 | $CF_2=CH$ | H | $CH_2F$ |
| 60 | $CF_2=CH$ | H | $C\equiv CH$ |
| 61 | $CF_2=CH$ | H | $CH_2OCOCH_3$ |

The compounds of the invention are esters and may be prepared by conventional esterification processes, of which the following are examples :

(a) An acid of formula (II)

X-COOH (II)

where X has any of the meanings given hereinabove, may be reacted directly with an alcohol of formula (III) :

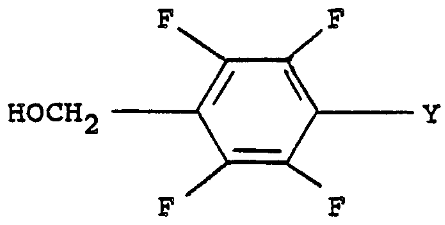

(III)

wherein Y has any of the meanings given hereinabove, the reaction preferably taking place in the presence of an acid catalyst, for example, dry hydrogen chloride, or a dehydrating agent, for example, a carbodiimide such as dicyclohexylcarbodiimide.

(b) An acid halide of formula X-COHal where Hal represents a halogen atom, preferably a chlorine atom, and X has any of the meanings given hereinabove, may be reacted with an alcohol of formula (III), the reaction preferably taking place in the presence of a base, for example, pyridine, a trialkylamine or an alkali metal hydroxide or carbonate.

(c) An acid of formula (II) where X has any of the meanings given hereinabove, or preferably, an alkali metal salt thereof, may be reacted with either (i) a compound of formula (IV) :

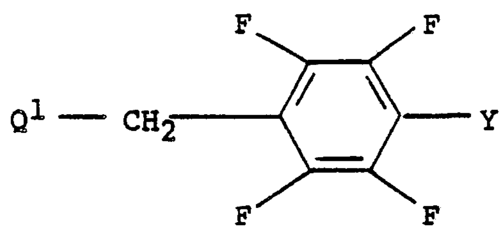

(IV)

where Y is as defined hereinabove and $Q^1$ represents a halogen atom, preferably the bromine or chlorine atom, or with a quaternary ammonium salt derived from such halides by reaction with a tertiary amine, for example pyridine or a trialkylamine such as triethylamine, or (ii) a compound of formula (IV) wherein $Q^1$ represents the mesylate or tosylate group.

(d) A lower alkyl ester of formula X-COOQ where Q represents a lower alkyl group containing up to six carbon atoms, preferably the methyl or ethyl group, and X has any of the meanings given hereinabove, is heated with an alcohol of formula (III) to effect a trans-esterification reaction. Preferably the process is performed in the presence of a suitable catalyst, for example, an alkali metal alkoxide such as sodium methoxide, or an alkylated titanium derivative such as tetramethyl titanate or tetraethyl titanate.

All of these conventional processes for the preparation of esters may be carried out using solvents and diluents for the various reactants where appropriate, and may be accelerated or lead to higher yields of product when performed at elevated temperatures or in the presence of appropriate catalysts, for example phase-transfer catalysts.

The preparation of individual isomers may be carried out in the same manner but commencing from the corresponding individual isomers of compounds of formula (II). These may be obtained by conventional isomer separation techniques from mixtures of isomers. Thus cis and trans isomers may be separated by fractional crystallisation of the carboxylic acids or salts thereof, whilst the various optically active species may be obtained by fractional crystallisation of salts of the acids with optically active amines, followed by regeneration of the optically pure acid. The optically pure isomeric form of the acid (or its equivalent acid chloride or ester) may then be reacted with the alcohol of formula (III) or a halide of formula (IV) to produce a compound of formula (I) in the form of an individually pure isomer thereof.

The alcohols of formula (III) and halides of formula (IV) may be prepared by means of a number of processes, the particular process selected in each case being dependent upon the nature of the group Y. Thus where Y represents $-CH_2F$, a halide of formula (IV) wherein Hal represents bromine may be prepared from 2,3,5,6-tetrafluoro-4-methylbenzyl alcohol by replacement of the hydroxy group using an appropriate fluorinating agent, for example diethylaminosulphur trifluoride, and conversion of the fluoromethyl derivative to 4-fluoromethyl-2,3,5,6-tetrafluorobenzyl bromide by reaction with a brominating agent such as N-bromosuccinimide.

Alcohols of formula (III) wherein Y represents $-CH_2CN$, $-CH_2N_3$ or $-CH_2OCHO$ may be prepared from 4-bromomethyl-2,3,5,6-tetrafluorobenzyl alcohol by nucleophilic substitution reactions as illustrated by way of example in Scheme I.

Scheme I

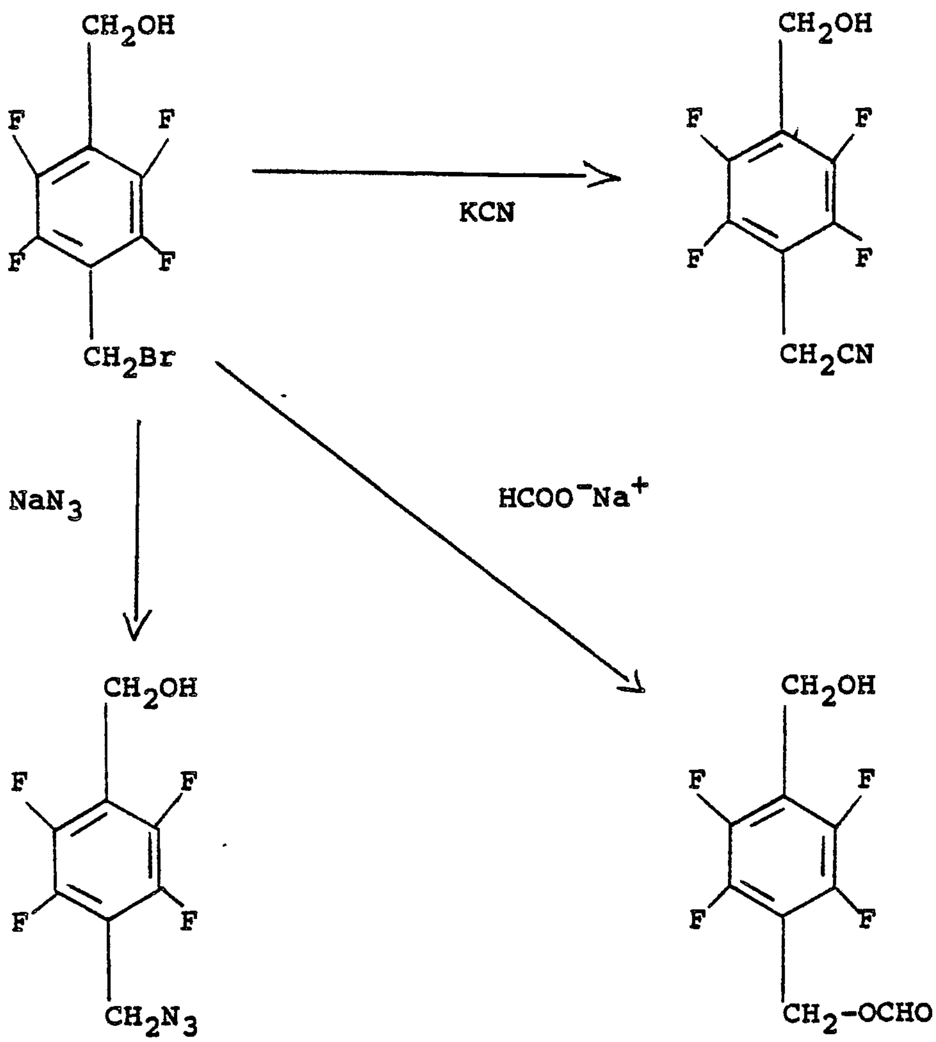


Alcohols of formula (III) wherein Y represents a group of formula $-NH-N=C(R^{11})R^{12}$ or a group of formula $-C\equiv CR^{10}$ may be prepared from pentafluorobenzyl alcohol by the processes described in Scheme II.

Scheme II

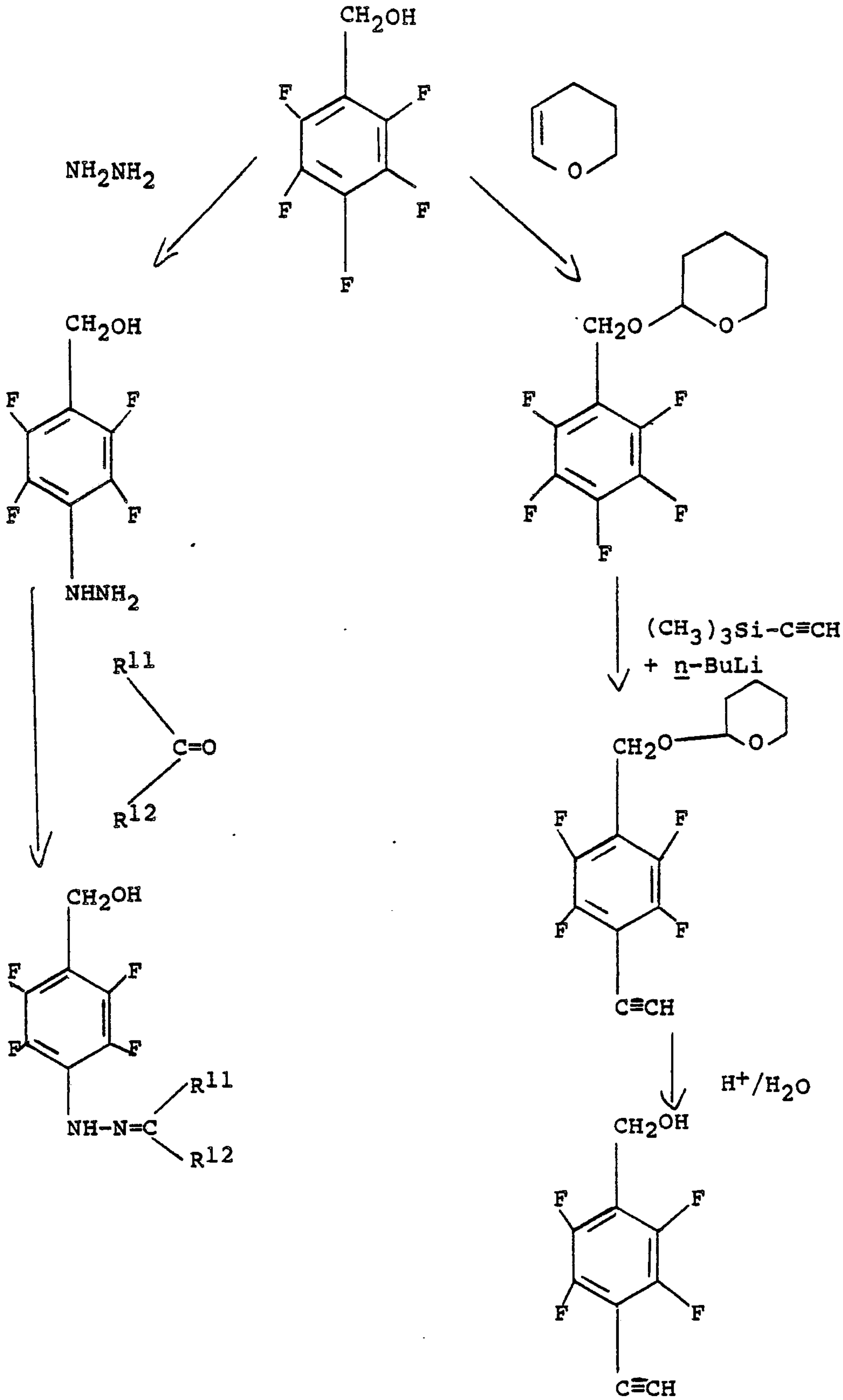

Alcohols of formula (III) wherein Y represents a group of formula $-CH_2SCOR^7$, $-CH_2OR^8$, $-CH_2SR^8$, $-CH_2N(R^{15})R^8$ or $-CH_2ON=C(R^{13})R^{14}$ may be readily prepared from 4-bromomethyl-2,3,5,6-tetrafluorobenzyl alcohol, or a derivative thereof in which the hydroxy group is protected by reaction with a suitable protecting agent, for example dihydropyran, by reaction with compounds of formula $R^7COSH$, $R^8OH$, $R^8SH$, $R^8(R^{15})NH$ and $R^{14}(R^{13})C=N-OH$ respectively. An alternative synthetic procedure for alcohols of formula (III) wherein Z represents the group $-SR^8$ is provided by reaction of 4-bromomethyl-2,3,5,6-tetrafluorobenzyl alcohol with thiourea to give 4-(hydroxymethyl)-2,3,5,6-tetrafluorobenzylmercaptan, which may then be reacted with compounds of formula $R^8$-Hal, wherein Hal represents a halogen atom, preferably chlorine or bromine.

4-(2-Cyanomethyl)-2,3,5,6-tetrafluorobenzyl alcohol may be prepared by a process as summarised in Scheme III :

Scheme III

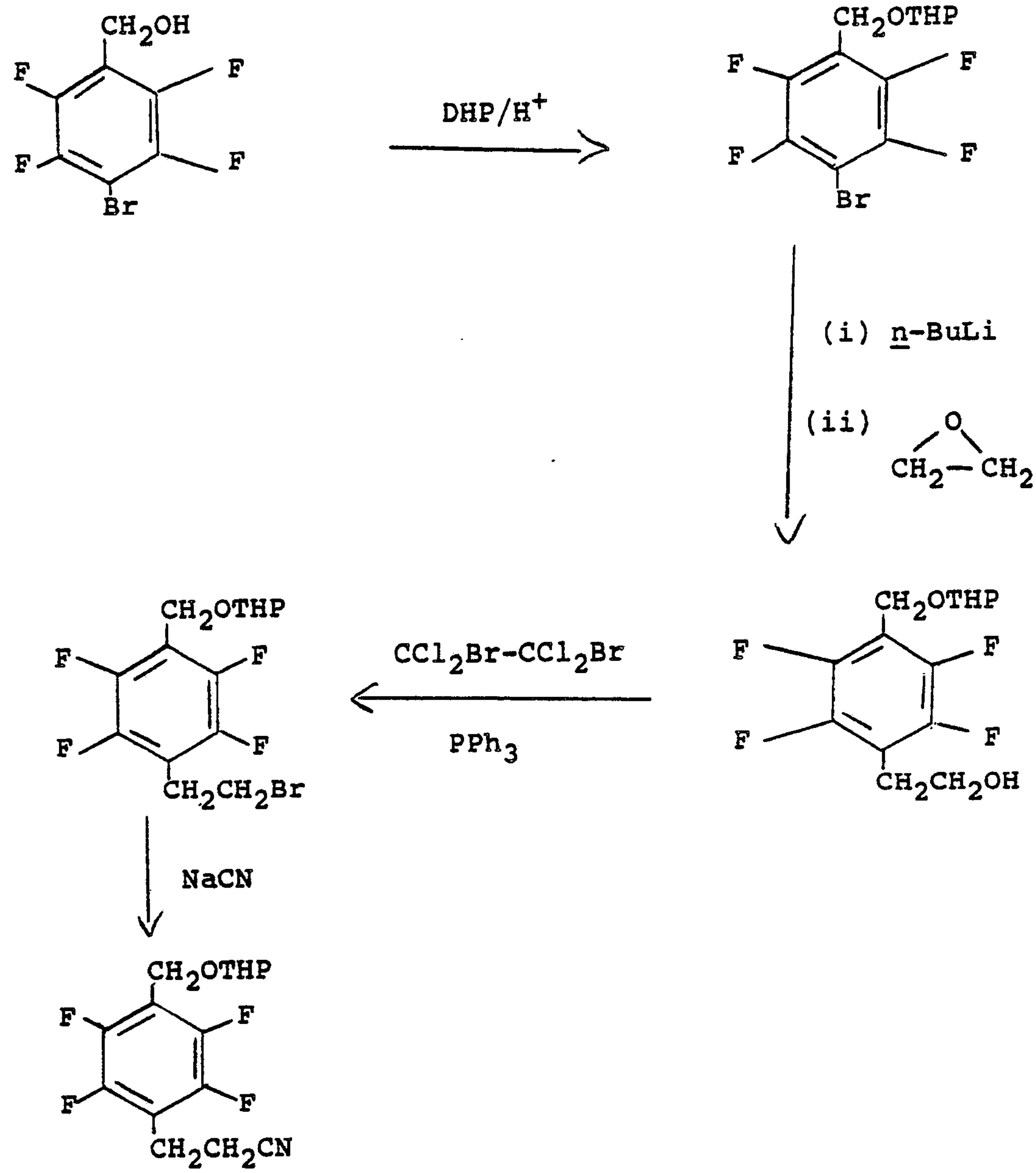


Key: DHP = dihydropyran

THP = tetrahydropyran

n-BuLi = n-butyllithium

$PPh_3$ = triphenylphosphine

Many of the compounds of formula (I) may be conveniently prepared from esters of formula (V) :

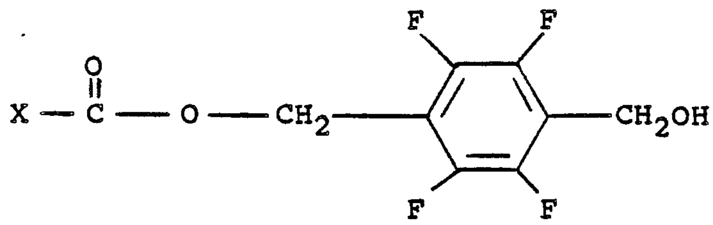

(V)

wherein X has any of the meanings given hereinabove.

Examples of suitable processes are illustrated in Scheme IV.

Scheme IV

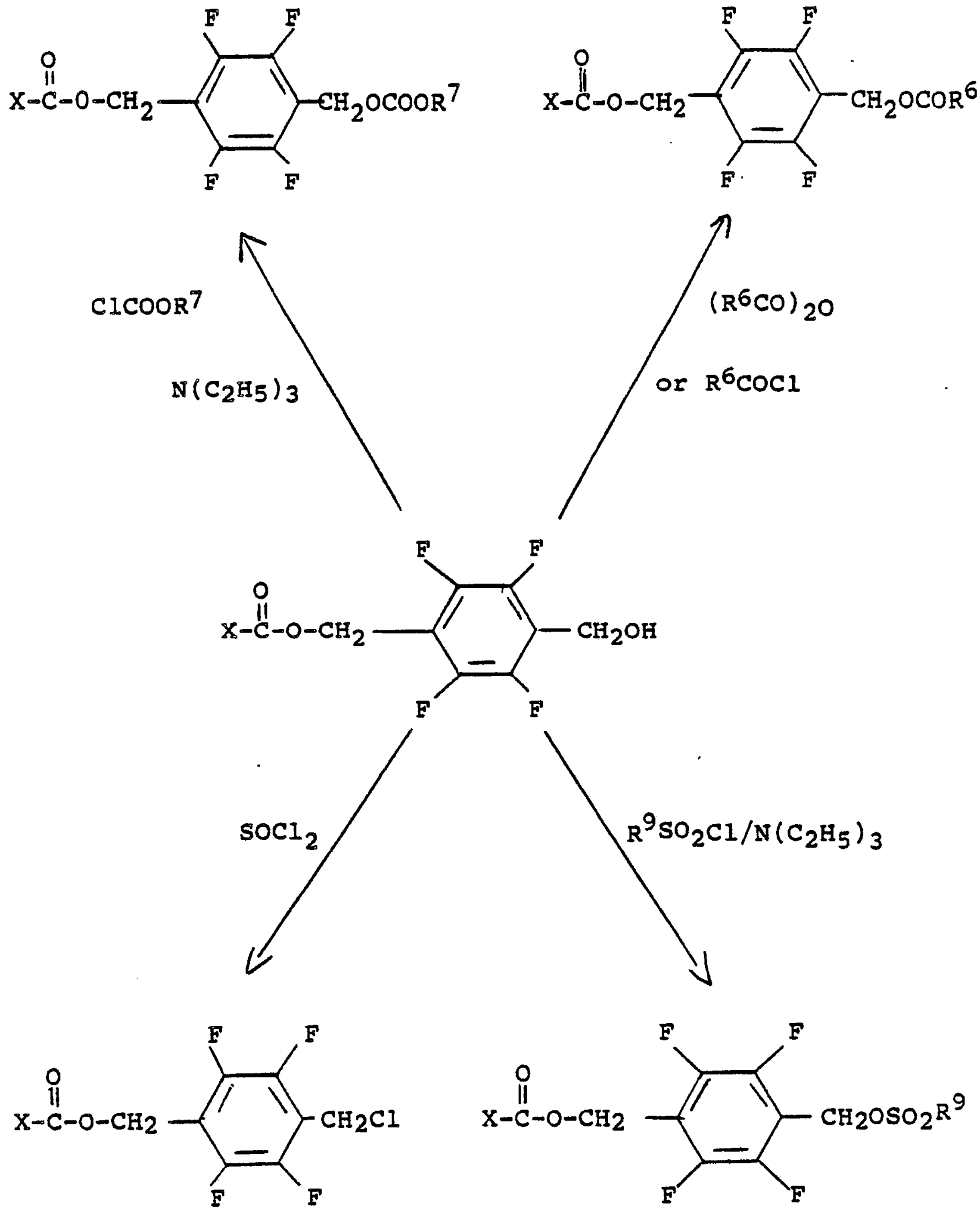


Many of the alcohols of formula (III) and the halides of formula (IV) have not been previously described. Accordingly, in a further aspect, the invention provides compounds of formula (V) :

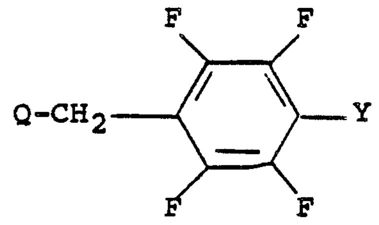

(V)

wherein Q represents hydroxy or halo and either :

(i) Y represents a group of formula $-CH_2-Z$ wherein Z is selected from fluoro, cyano, azido and cyanomethyl, or Z is selected from a group of formula $-OCOR^6$, a group of formula $-SCOR^7$, a group of formula $-OCO_2R^7$, a group of $-OR^8$, a group of formula $-SR^8$, a group of formula $-N(R^{15})R^8$, a group of formula $-OSO_2R^9$, and a group of formula $-ON=C(R^{13})R^{14}$, wherein $R^6$ represents hydrogen, $C_{1-4}$ alkyl or $C_{1-4}$ haloalkyl, $R^8$ represents propargyl optionally substituted with one or more methyl groups, $R^7$ and $R^9$ represent $C_{1-4}$ alkyl or $C_{1-4}$ haloalkyl, and $R^{13}$, $R^{14}$ and $R^{15}$ represent $C_{1-4}$ alkyl, or

(ii) Y represents a group of formula $-C\equiv CR^{10}$ or a group of formula $-NH-N=C(R^{11})R^{12}$ wherein $R^{10}$, $R^{11}$ and $R^{12}$ are selected from hydrogen, $C_{1-4}$ alkyl and $C_{1-4}$ haloalkyl.

Details of the preparation of the compounds of formula (III) and of other processes described hereinbefore may be ascertained from the specific examples set forth hereinafter.

The compounds of formula (I) may be used to combat and control infestations of insect and acarine pests. The insect and acarine pests which may be combated and controlled by the use of the invention compounds include those pests associated with agriculture (which term includes the growing of crops for food and fibre products, horticulture and animal husbandry), forestry, the storage of products of vegetable origin, such as fruit, grain and timber, and also those pests associated with the transmission of diseases of man and animals.

In order to apply the compounds to the locus of the pests they are usually formulated into compositions which include in addition to the insecticidally active ingredient or ingredients of formula (I) suitable inert diluent or carrier material, and/or surface active agents.

The compounds of the invention may be the sole active ingredient of the composition or they may be admixed with one or more additional active ingredients such as insecticides, insecticide synergist, herbicides, fungicides or plant growth regulators where appropriate.

Suitable additional active ingredients for inclusion in admixture with the compounds of the invention may be compounds which will broaden the spectrum of activity of the compounds of the invention or increase their persistence in the location of the pest. They may synergise the activity of the compounds of the invention or complement the activity for example by increasing the speed of effect, improving knockdown or overcoming repellency. Additionally multi-component mixtures of this type may help to overcome or prevent the development of resistance to individual components.

The particular insecticide, herbicide or fungicide included in the mixture will depend upon its intended utility and the type of complementary action required. Examples of suitable insecticides include the following:

(a) Nature pyrethrins and pyrethroids such as resmethrin, permethrin, fenvalerate, deltamethrin, cyhalothrin, biphenthrin, fenpropathrin, cyfluthrin, tefluthrin, empenthrin, fish safe pyrethroids for example ethofenprox, tetramethrin, allethrin, fenfluthrin, prallethrin, 5-benzyl-3-furylmethyl-(E)-(1R,3S)-2,2-dimethyl-3-(2-oxothiolan-3-ylidenemethyl)cyclopropane carboxylate and penta-fluorobenzyl (cis)-3-(2-fluoro-2-methoxycarbonyl)-2,2-dimethylcyclopropanecarboxylate;

(b) Organophosphates such as profenofos, sulprofos, dichlorvos, methyl parathion, azinphos-methyl, demeton-s-methyl, heptenophos, thiometon, fenamiphos, monocrotophos, profenophos, triazophos, methamidophos, dimethoate, phosphamidon, malathion, chlorpyrifos, phosalone, fensulfothion, fonofos, phorate, phoxim, pyrimiphos-methyl, fenitrothion and diazinon;

(c) Carbamates (including aryl carbamates) such as pirimicarb, cloethocarb, carbofuran, ethiofencarb, aldicarb, thiofurox, carbosulfan, bendiocarb, fenobucarb, propoxur and oxamyl;

(d) Benzoyl ureas such as triflumuron, chlorfluazuron;

(e) Organic tin compounds such as cyhexatin, fenbutatin oxide, azocyclotin;

(f) Macrolides such as avermectins or milbemycins, for example such as abamectin, avermectin, and milbemycin;

(g) Hormones and synthetic mimics thereof such as juvenile hormone, juvabione, ecdysones, methoprene and hydroprene.

(h) Pheromones.

(i) Organochlorine compounds such as benzene hexachloride, DDT, chlordane or dieldrin.

In addition to the major chemical classes of insecticide listed above, other insecticides having particular targets may be employed in the mixture if appropriate for the intended utility of the mixture. For instance selective insecticides for particular crops, for example stemborer specific insecticides for use in rice such as cartap or buprofezin, can be employed. Alternatively insecticides or acaricides specific for particular insect species/stages for example ovolarvicides such as clofentezine, amitraz, chlordimeform, flubenzimine, hexythiazox and tetradifon, motilicides such as dicofol or propargite, adulticides such as bromopropylate, chlorobenzilate, or insect growth regulators such as hydramethylnon, cyromazine, methoprene, chlorfluazuron and diflubenzuron may also be included in the compositions.

Examples of suitable insecticide synergists for use in the compositions include piperonyl butoxide, sesamex, and dodecyl imidazole.

Suitable herbicides, fungicides and plant growth regulators for inclusion in the compositions will depend upon the intended target and the effect required. An example of a rice selective herbicide which can be included is propanil, an example of a plant growth regulator for use in cotton is "Pix", and examples of fungicides for use in rice include blasticides such as blasticidin-S. The choice of other ingredients to be used in mixture with the active ingredient will often be within the normal skill of the formulator, and will be made from known alternatives depending upon the total effect to be achieved.

The ratio of the compound of the invention to any other active ingredient in the composition will depend upon a number of factors including the type of insect pests to be controlled, and the effects required from the mixture. However in general, the additional active ingredient of the composition will be applied at about the rate it would usually be employed if used on its own, or at a lower rate if synergism occurs.

The compositions may be in the form of dusting powders wherein the active ingredient is mixed with a solid diluent or carrier, for example kaolin, bentonite, kieselguhr, or talc, or they may be in the form of granules, wherein the active ingredient is absorbed in a porous granular material, for example pumice.

Alternatively the compositions may be in the form of liquid preparations to be used as dips, sprays or aerosols. Dips and sprays are generally aqueous dispersions or emulsions of the active ingredient in the presence of one or more known wetting agents, dispersing agents or emulsifying agents (surface active agents). Aerosol compositions may contain the active ingredient or ingredients, a propellant and an inert diluent, for example odourless kerosene or alkylated benzenes. In a preferred form, aerosol compositions may contain from 0.005% to 4% of active ingredient or ingredients, the remainder of the composition comprising a solvent, selected from odourless kerosene and alkylated benzenes, and a propellant. Aerosol compositions may optionally incorporate other additives, for example perfumes or corrosion inhibitors.

Wetting agents, dispersing agents and emulsifying agents may be of the cationic, anionic or non-ionic type. Suitable agents of the cationic type include, for example, quaternary ammonium compounds, for example cetyltrimethyl ammonium bromide. Suitable agents of the anionic type include, for example, soaps, salts of aliphatic monoesters or sulphuric acid, for example sodium lauryl sulphate, salts of sulphonated aromatic compounds, for example sodium dodecylbenzenesulphonate, sodium, calcium or ammonium lignosulphonate, or butylnaphthalene sulphonate, and a mixture of the sodium salts of diisopropyl- and triisopropylnaphthalene sulphonates. Suitable agents of the non-ionic type include, for example, the condensation products of ethylene oxide with fatty alcohols such as oleyl alcohol or cetyl alcohol, or with alkyl phenols such as octyl phenol, nonyl phenol and octyl cresol. Other non-ionic agents are the partial esters derived from long chain fatty acids and hexitol anhydrides, the condensation products of the said partial esters with ethylene oxide, and the lecithins.

The compositions may be prepared by dissolving the active ingredient in a suitable solvent, for example, a ketonic solvent such as diacetone alcohol, or an aromatic solvent such as trimethylbenzene and optionally adding the mixture so obtained to water which may contain one or more known wetting, dispersing or emulsifying agents.

Other suitable organic solvents are dimethyl formamide, ethylene dichloride, isopropyl alcohol, propylene glycol and other glycols, diacetone alcohol, toluene, kerosene, white oil, methylnaphthalene, xylenes and trichloroethylene, N-methyl-2-pyrrolidone and tetrahydrofurfuryl alcohol (THFA).

The compositions which are to be used in the form of aqueous dispersions or emulsions are generally supplied in the form of a concentrate containing a high proportion of the active ingredient or ingredients, the said concentrate to be diluted with water before use. These concentrates are often required to withstand

storage for prolonged periods and after such storage, to be capable of dilution with water to form aqueous preparations which remain homogenous for a sufficient time to enable them to be applied by conventional spray equipment. The concentrates may contain 1-99% by weight of the active ingredient or ingredients. When diluted to form aqueous preparations such preparations may contain varying amounts of the active ingredient depending upon the purpose for which they are to be used. For agricultural or horticultural purposes, an aqueous preparation containing between 0.0001% and 0.1% by weight of the active ingredient is particularly useful.

In use the compositions are applied to the pests, to the locus of the pests, to the habitat of the pests, or to growing plants liable to infestation by the pests, by any of the known means of applying pesticidal compositions, for example, by dusting or spraying.

The compounds of formula (I) and compositions comprising them are very toxic to wide varieties of insect, acarine and other invertebrate pests, including, for example, the following :

Myzus persicae (aphids)
Aphis gossypii (aphids)
Aphis fabae (aphids)
Megoura viceae (aphids)
Aedes aegypti (mosquitos)
Anopheles spp. (mosquitos)
Culex spp. (mosquitos)
Dysdercus fasciatus (capsids)
Musca domestica (houseflies)
Pieris brassicae (white butterfly, larvae)
Plutella maculipennis (diamond back moth, larvae)
Phaedon cochleariae (mustard beetle)
Aonidiella spp. (scale insects)
Trialeuroides spp. (white flies)
Bemisia tabaci (white flies)
Blattella germanica (cockroaches)
Periplaneta americana (cockroaches)
Blatta orientalis (cockroaches)
Spodoptera littoralis (cotton leaf worm)
Heliothis virescens (tobacco budworms)
Chortiocetes terminifera (locusts)
Diabrotica spp. (rootworms)
Agrotis spp. (cutworms)
Chilo partellus (maize stem borers)
Nilaparvata lugens (plant hoppers)
Nephotettix cincticeps (leaf hoppers)
Panonychus ulmi (European red mite)
Panonychus citri (citrus red mite)
Tetranychus urticae (two-spotted spider mite)
Tetranychus cinnabarinus (carmine spider mite)
Phyllocoptruta oleivora (citrus rust mite)
Polyphagotarsonemus latus (broad mite)
Brevipalpus spp. (mites)

The compounds according to formula (I) and compositions comprising them have been shown to be particularly useful in controlling lepidopteran pests of cotton, for example Spodoptera spp. and Heliothis spp. They have also been shown to be particularly useful in combating pests which inhabit the soil, for example Diabrotica spp. Many of the compounds of formula (I) have also been shown to exhibit high levels of knockdown activity against public health insect pests such as Musca domestica (housefly), Blattella germanica (cockroach) and Aedes aegypti (mosquito). They may also be useful in combating insect and acarine pests which infest domestic animals, such as Lucilia sericata an ixodid ticks such as d Boophilus spp., Ixodes spp., Amblyomm spp., Rhipicephalus a spp., and Dermaceutor spp. They are effective in combating both susceptible and resistant strains of these pests in their adult, larval and intermediate stages of growth, and may be applied to the infested host animal by topical, oral or parental administration.

The following Examples illustrate various aspects of this invention. In the preparation Examples the products were usually identified and characterised by means of nuclear magnetc resonance (NMR) spectroscopy and infra red (IR) spectroscopy. In each case where a product is specifically named its

spectral characteristics are consistent with the assigned structure. Except where stated otherwise, exemplified compounds having one or more asymmetrically substituted carbon atoms were prepared in racemic form.

In the Examples, gas liquid chromatography (GLC) retention times were determined on a Hewlett Packard 5890 Gas Chromatograph, using a Chrompak C.P. Sil 5 C.B. column of 12.5m length and 0.2mm internal diameter. Unless otherwise stated, the injection temperature was 100 °C, and a temperature gradient of 15 °C per minute employed, up to a maximum temperature of 280 °C, maintained for 4 minutes. The carrier gas was helium at a column head pressure maintained at 11 psi. Alternative injection and maximum temperatures are indicated in the Examples where appropriate.

$^1H$ NMR spectrometry was performed at a frequency of 270 MHz on a Jeol FX 270 NMR spectrometer unless otherwise indicated. 90 MHZ, 60 MHZ, and 400 MHz $^1H$ NMR spectrometry was performed using Jeol FX 90Q, Jeol PMX 60 SI, and Jeol GX 400 spectrometers. $^{19}F$ NMR spectrometry was performed using a Jeol FX 90Q spectrometer at a frequency of 84.26 MHZ. All NMR shift values (δ) are quoted in ppm relative to a standard (TMS or $CFCl_3$). In the NMR data, the following abbreviations are used: s = singlet, d = doublet, t = triplet, q = quartet, dd = double doublet, m = multiplet, b = broad.

Molecular ion ($M^+$) peaks were determined on one of three mass spectrometers: Jeol DX 303, Kratos MS 80 or Hewlett Packard HP 5992.

## EXAMPLE 1

This Example illustrates the stages in the preparation of 4-(fluoromethyl)-2,3,5,6-tetrafluorobenzyl bromide.

### STAGE I : Preparation of 4-(fluoromethyl)-2,3,5,6-tetrafluorotoluene

A stirred solution of 4-methyl-2,3,5,6-tetrafluorobenzyl alcohol (3g, prepared according to the method described in UK patent application No. 2066810) in dichloromethane (20 $cm^3$) maintained under an atmosphere of nitrogen was cooled to -10 °C and N,N-diethylaminosulphurtrifluoride (3.0 $cm^3$) was added dropwise. The temperature of the mixture was maintained below 0 °C during the addition and for a further 30 minutes. The mixture was allowed to warm to the ambient temperature (ca 25 °C) and stirred for 3 hours before being poured into water. The products were extracted into dichloromethane. The organic layers were combined and washed with sodium bicarbonate solution, then dried over anhydrous magnesium sulphate. Evaporation of the solvent under reduced pressure gave an oil which was purified by flash column chromatography on a silica gel support, eluting with dichloromethane. Evaporation of the eluent gave a liquid which was further purified by short path distillation at a bulb temperature of 50 °C under reduced pressure (0.5 mbar) to give 4-(fluoromethyl)-2,3,5,6-tetrafluorotoluene (1g) as a colourless liquid.

$^1H$ NMR ($CDCl_3$) : 5.5 (2H,d); 2.3 (3H,s)

$^{19}F$ NMR ($CDCl_3$) : -211 (t); -144 (d).

### STAGE 2 : Preparation of 4-(fluoromethyl)-2,3,5,6-tetrafluorobenzyl bromide

A stirred mixture of 4-fluoromethyl-2,3,5,6-tetrafluorotoluene (1.0g), N-bromosuccinimide (0.95g) and dibenzoyl peroxide (catalytic quantity) in carbon tetrachloride (15 $cm^3$) was heated at the reflux temperature under illumination from a tungsten lamp for 3.5 hours. After cooling, the mixture was filtered and the filtrate concentrated by evaporation under reduced pressure. The resultant oil was purified by flash column chromatography on a silica gel support, eluting with hexane containing 5% by volume diethyl ether to give 4-(fluoromethyl)-2,3,5,6-tetrafluorobenzyl bromide (0.8g) as a colourless liquid.

$^1H$ NMR ($CDCl_3$) : 5.5 (2H, d); 4.5 (2H, s).

$^{19}F$ NMR ($CDCl_3$) : -214 (t); -144 (d).

## EXAMPLE 2

This Example illustrates the preparation of 4-(bromomethyl)-2,3,5,6-tetrafluorobenzyl bromide.

STAGE 1 : Preparation of 4-methyl-2,3,5,6-tetrafluorotoluene

A solution of n-butyllithium (95 $cm^3$ of a 1.6 molar solution in hexane) was added dropwise to a stirred solution of 2,3,5,6-tetrafluorotoluene (25g) in dry tetrahydrofuran (75 $cm^3$) at -70°C under a nitrogen atmosphere and the mixture stirred for 2 hours.

Methyl iodide (21.9g) was added dropwise over a period of 30 minutes. After the mixture had been allowed to warm to the ambient temperature of 25°C it was poured into water, and the product was extracted into diethyl ether. The ethereal extract was washed with water and dried over anhydrous magnesium sulphate. Removal of the solvent by evaporation under reduced pressure yielded a yellow oil (23.5g) which was distilled at 100°C under reduced pressure (100mmHg) to yield 4-methyl-2,3,5,6-tetrafluorotoluene (16.5g) as a colourless liquid.

$^1H$ NMR ($CDCl_3$) : 2.3 (s).

STAGE 2 : Preparation of 4-(bromomethyl)-2,3,5,6-tetrafluorobenzyl bromide

A stirred solution of 4-methyl-2,3,5,6-tetrafluorotoluene (16.5g) and n-bromosuccinimide (33.0g) in carbon tetrachloride (450 $cm^3$) was heated at the reflux temperature for 7 hours under irradiation from an ultra violent lamp. The mixture was cooled to the ambient temperature and the solid suspension removed by filtration. The filtrate was concentrated by evaporation under reduced pressure to yield a solid which was recrystallised from petroleum ether (boiling range 60-80°C) to yield the title compound as a white solid (16.5g).

$^1H$ NMR ($CDCl_3$) : 4.8 (s).

## EXAMPLE 3

This Example illustrates the preparation of 4-(hydroxymethyl)-2,3,5,6-tetrafluorobenzyl (1RS,cis)-3-(Z-2-chloro-3,3,3-trifluoroprop-1-en-1-yl)-2,2-dimethylcyclopropane carboxylate.

A mixture of 4-(hydroxymethyl)-2,3,5,6-tetrafluorobenzylbromide (1.3g, prepared according to the method described in UK patent application No. 2153819A), (1RS,cis-3-(Z-2-chloro-3,3,3-trifluoroprop-1-en-1-yl)-2,2-dimethylcyclopropanecarboxylic acid (1.5g), potassium carbonate (1.4g) and ethylmethylketone (50 $cm^3$) was heated at the reflux temperature for 30 minutes. After cooling, the mixture was poured into water and the products extracted into diethyl ether (2 x 50 $cm^3$). The combined ethereal extracts were washed with water and dried over anhydrous magnesium sulphate. Evaporation of the solvent under reduced pressure gave an oil which was purified by flash column chromatography on a silica gel support, eluting with dichloromethane, to give the title compound 2.1g) as a colourless oil.

400 MHz $^1H$ NMR ($CDCl_3$) : 6.9 (1H, d); 5.23 (2H, q); 4.85 (2H, d); 2.2 (2H, t); 1.95 (1H, d); 1.30 (6H, s).

## EXAMPLE 4

The following compounds were prepared from the appropriate starting materials by a method similar to that described in Example 3.

(i) 4-(Bromomethyl)-2,3,5,6-tetrafluorobenzyl (1RS,cis)-3-(Z-2-chloro-3,3,3-trifluoroprop-1-en-1-yl)-2,2-dimethyl-cyclopropanecarboxylate, (Product I)

Note: in this case the mixture was stirred at the ambient temperature for 17 hours.

$^1H$ NMR ($CDCl_3$) : 6.90 (1H, d); 5.24 (2H, q); 4.54 (2H, s); 2.20 (1H, t); 1.98 (1H, d); 1.30 (6H, s).

(ii) 4-(Fluoromethyl)-2,3,5,6-tetrafluorobenzyl (1RS,cis)-3-(Z-2-chloro-3,3,3-trifluoroprop-1-en-1-yl)-2,2-dimethyl-cyclopropanecarboxylate, (Product II)

$^1H$ NMR ($CDCl_3$) : 6.85 (1H,d); 5.80 (1H,broad s); 5.23 (3H, broad s); 1.9-2.3 (2H,m); 1.30 (6H,s)

## EXAMPLE 5

This Example illustrates the preparation of 4-(chloromethyl)-2,3,5,6-tetrafluorobenzyl (1RS,cis)-3-(Z-2-chloro-3,3,3-trifluoroprop-1-en-1-yl)-2,2-dimethylcyclopropanecarboxylate, (Product III).

Thionyl chloride (0.1g) was added to a stirred solution of 4-(hydroxymethyl)-2,3,5,6-tetrafluorobenzyl (1RS,cis)-3-(Z-2-chloro-3,3,3-trifluoroprop-1-en-1-yl)-2,2-dimethylcyclopropanecarboxylate (0.217g) in dry diethyl ether (5 $cm^3$) and the mixture heated at the reflux temperature for 6 hours. After cooling, the solvent was evaporated under reduced pressure and the residual oil was purified by flash column chromatography, eluting with dichloromethane, to give the title compound (0.12g) as a colourless oil.

$^1H$ NMR ($CDCl_3$) : 6.90 (1H, d); 5.24 (2H, q); 4.84 (2H, s); 2.20 (1H, t); 1.98 (1H, d); 1.30 (6H, s).

## EXAMPLE 6

This Example illustrates the preparation of 4-(methanesulphonyloxymethyl)-2,3,5,6-tetrafluorobenzyl (1RS,cis)-3-(Z-2-chloro-3,3,3-trifluoroprop-1-en-1-yl)-2,2-dimethylcyclopropanecarboxylate, (Product IV)

A mixture of 4-(hydroxymethyl)-2,3,5,6-tetrafluorobenzyl (1RS,cis)-3-(Z-2-chloro-3,3,3-trifluoroprop-1-en-1-yl)-2,2-dimethylcyclopropane carboxylate (0.217g), triethylamine (0.06g) and diethyl ether (5 $cm^3$) was stirred at the ambient temperature (ca. 20 °C). Methanesulphonyl chloride (0.06g) was added to the mixture, and stirring continued for one hour.

The mixture was filtered and the filtrate concentrated by evaporation of the solvent under reduced pressure. The residual oil was purified by flash column chromatography on a silica gel support, eluting with dichloromethane to give the title compound as a colourless oil (0.16g).

60 MHz $^1H$ NMR ($CDCl_3$) : 6.90 (1H, d); 5.36 (2H, s); 5.24 (2H, s); 3.1 (3H, s); 1.9-2.4 (1H, m); 1.3 (6H, s).

## EXAMPLE 7

This Example illustrates the preparation of 4-(trifluoroacetoxymethyl)-2,3,5,6-tetrafluorobenzyl (1RS,cis)-3-(Z-2-chloro-3,3,3-trifluoroprop-1-en-1-yl)-2,2-dimethylcyclopropanecarboxylate, (Product V)

Trifluoroacetic anhydride (0.1g) was added to a stirred solution of 4-(hydroxymethyl)-2,3,5,6-tetrafluorobenzyl (1RS,cis)-3-(Z-2-chloro-3,3,3-trifluoroprop-1-en-1-yl)-2,2-dimethylcyclopropanecarboxylate (0.217g) in diethyl ether (3 $cm^3$) at the ambient temperature (ca. 20 °C). Stirring was continued for a further hour and volatile components were then removed by evaporation under reduced pressure. The residual oil was purified by flash column chromatography on a silica gel support, eluting with dichloromethane, to give the title compound (0.16g) as a colourless oil.

$^1H$ NMR ($CDCl_3$) : 6.86 (1H, d); 5.50 (2H, s); 5.26 (2H, q); 2.20 (1H, t); 1.99 (1H, d); 1.30 (6H, s).

Infra red (liquid film) : 1800, 1740 $cm^{-1}$.

EXAMPLE 8

The following compounds were prepared by reaction of 4-(hydroxymethyl)-2,3,5,6-tetrafluorobenzyl (1RS,cis)-3-(Z-2-chloro-3,3,3-trifluoroprop-1-en-1-yl)-2,2,-dimethylcyclopropanecarboxylate with the appropriate starting material according to the method described in Example 6.

(i) 4-(Acetoxymethyl)-2,3,5,6-tetrafluorobenzyl (1RS,cis)-3-(Z-2-chloro-3,3,3-trifluoroprop-1-en-1-yl)-2,2-dimethylcyclopropanecarboxylate (Product VI) by reaction with acetyl chloride.

Note: the reaction temperature in this case was maintained at 0°C.

$^1H$ NMR ($CDCl_3$) : 6.86 (1H, d); 5.24 (4H, m); 2.19 (1H, t); 2.09 (3H, s); 1.96 (1H, d); 1.30 (6H, s).

(ii) 4-(Methoxycarbonyloxymethyl)-2,3,5,6-tetrafluorobenzyl (1RS,cis)-3-(Z-2-chloro-3,3,3-trifluoroprop-1-en-1-yl)-2,2-dimethylcyclopropanecarboxylate (Product VII), by reaction with methyl chloroformate.

400 MHz$^1$ H NMR ($CDCl_3$) : 6.86 (1H,d); 5.15-5.35 (4H,m); 3.83 (3H,s); 2.22 (2H,t); 1.98 (1H,d); 1.30 (6H,s).

Infra red (liquid film) : 1760, 1730 $cm^{-1}$.

EXAMPLE 9

This Example illustrates the stages in the preparation of 4-ethynyl-2,3,5,6-tetrafluorobenzyl alcohol.

STAGE 1 : Preparation of pentafluorobenzyl tetrahydropyran-2-yl ether

A mixture of pentafluorobenzyl alcohol (17.87g), 2,3-dihydropyran (8.3g), diethyl ether (100 $cm^3$) and concentrated aqueous hydrochloric acid solution (0.2 $cm^3$) was stirred at the ambient temperature (ca. 20°C) for 4 hours, after which time the mixture was washed with water, dried over anhydrous magnesium sulphate and concentrated by evaporation under reduced pressure. The residual oil was identified by infra red spectroscopy as pentafluorobenzyl tetrahydropyran-2-yl ether.

STAGE 2 : Preparation of 4-ethynyl-2,3,5,6-tetrafluorobenzyl tetrahydropyran-2-yl ether

A solution of trimethylsilylacetylene (3.6g) in tetrahydrofuran (10 $cm^3$) was added dropwise to a stirred solution of n-butyllithium (15 $cm^3$ of a 2.5 molar solution in hexane) in tetrahydrofuran (10 $cm^3$) at -70°C under an atmosphere of nitrogen. The mixture was stirred for 30 minutes. A solution of pentafluorobenzyl tetrahydropyran-2-yl ether (5.0g) in tetrahydrofuran (10 $cm^3$) was added to the cooled reaction mixture and the temperature allowed to rise to -20°C; the mixture was stirred at this temperature for 30 minutes and then at the ambient temperature for 3 hours. The mixture was again cooled to -70°C and further trimethylsilylacetylene (3.4g) was added, under nitrogen. n-Butyllithium (15 $cm^3$ of a 2.5 M solution in hexane) was added dropwise, keeping the temperature at ca. -70°C. The mixture was allowed to warm to the ambient temperature.

The mixture was poured into water and the products extracted into diethyl ether. The combined ethereal extracts were dried over anhydrous magnesium sulphate and the solvent evaporated under reduced pressure to leave an oil (6.8g) which was purified by high pressure liquid chromatography, eluting with hexane containing 5% by volume diethyl ether. Two products were detected, the slower running product being identified as 4-ethynyl-2,3,5,6-tetrafluorobenzyl tetrahydropyran-2-yl ether (0.6g).

STAGE 3 : Preparation of 4-ethynyl-2,3,5,6-tetrafluorobenzyl alcohol

A mixture of 4-ethynyl-2,3,5,6-tetrafluorobenzyl tetrahydropyran-2-yl ether (0.6g), methanol (3 $cm^3$) and dilute aqueous hydrochloric acid (1 $cm^3$, 2 molar) was stirred at the ambient temperature for 2 hours. The mixture was poured into water and the products extracted into diethyl ether. The combined ethereal layers were washed with water and dried over anhydrous magnesium sulphate. Evaporation of the solvent under reduced pressure gave an oil which was purified by column chromatography on a silica gel support, eluting with dichloromethane, to give the title compound (0.4g) as a colourless oil.

$^1$H NMR ($CDCl_3$) : 4.81 (2H,d); 3.62 (1H,s); 2.00 (1H,t).

EXAMPLE 10

This Example illustrates the preparation of 4-(cyanomethyl)-2,3,5,6-tetrafluorobenzyl alcohol.

A solution of potassium cyanide (0.5g) in dimethyl sulphoxide (8 $cm^3$) was added to a stirred solution of 4-(hydroxymethyl)-2,3,5,6-tetrafluorobenzyl bromide (0.35g prepared according to the method described in UK Patent Application No. 2153819A) in dimethyl sulphoxide (7 $cm^3$) at the ambient temperature. The mixture was stirred for a further 30 minutes then poured into water. The products were extracted into diethyl ether and the ethereal layers washed with water and dried over anhydrous magnesium sulphate. Evaporation of the solvent under reduced pressure gave an oil which was purified by flash column chromatography on a silica gel support, eluting with hexane containing 50% by volume diethyl ether, to give the title compound as a solid (0.12g).

Melting point : 81-82°C

60 MHz $^1$H NMR ($CDCl_3$) : 4.84 (2H, s); 3.80 (2H, s); 2.12 (1H, s).

EXAMPLE 11

This Example illustrates the preparation of 4-(azidomethyl)-2,3,5,6-tetrafluorobenzyl alcohol.

A solution of sodium azide (1.5g) in water (3 $cm^3$) was added to a stirred solution of 4-(hydroxymethyl)-2,3,5,6-tetrafluorobenzyl bromide (1.0g) and tetra-n-butylammonium iodide (catalytic amount) in dichloromethane (3 $cm^3$) and vigorous stirring continued for 2 hours. The layers were separated and the organic layer passed through phase separation paper to remove residual water. Evaporation of the solvent under reduced pressure yielded an oil which was purified by flash column chromatography on a silica gel column, eluting with dichloromethane, to give the title compound as a crystalline solid (0.8g).

$^1$H NMR ($CDCl_3$) : 4.83 (2H, s); 4.43 (2H, s); 2.6 (1H, s).

Infra red (paraffin mull) : 3400, 2100 $cm^{-1}$.

EXAMPLE 12

This Example illustrates the preparation of 4-(formyl-oxymethyl)-2,3,5,6-tetrafluorobenzyl alcohol.

A solution of sodium formate (0.5g) in water (3 $cm^3$) was added to a stirred solution of 4-(hydroxymethyl)-2,3,-5,6-tetrafluorobenzyl bromide (0.27g) and tetra-n-butylammonium iodide (catalytic

quantity) in dichloromethane (3 $cm^3$) at the ambient temperature. Stirring was continued for 2 hours. The organic layer was separated and dried by passing through phase separation paper. Evaporation of the solvent under reduced pressure gave an oil which was purified by flash column chromatography on a silica gel support, eluting with dichloromethane, to give the title compound as an oil (0.1g).

$^1H$ NMR ($CDCl_3$) : 7.98 (1H, s); 5.22 (2H, s); 4.72 (2H, d); 2.8 (1H, t).

EXAMPLE 13

This Example illustrates the preparation of 4-hydrazino-2,3,5,6-tetrafluorobenzyl alcohol.

A mixture of pentafluorobenzyl alcohol (18.1g) and hydrazine hydrate (10 $cm^3$) was heated to 100°C and stirred at this temperature for 17 hours. The reaction was cooled to the ambient temperature and added to ethyl acetate. The ethyl acetate mixture was then washed with water (2 x 100 $cm^3$) and brine (2 x 50 $cm^3$) and dried over anhydrous magnesium sulphate. The solvent was evaporated under reduced pressure to give a solid which was triturated with diethyl ether. The solid was collected by filtration, dried and identified as the title compound (14.0g).

Melting point : 103-105°C.

90 MHz $^1H$ NMR ($CDCl_3$) : 5.75 (1H, broad s); 4.70 (2H, s; 4.8-3.8 (3H, broad s).

$^{19}F$ NMR ($CDCl_3$) : -147.2 to -147.62; -159.5 to -160.0.

EXAMPLE 14

This Example illustrates the preparation of 4-(prop-2-ylidenehydrazino)-2,3,5,6-tetrafluorobenzyl alcohol.

Aqueous hydrochloric acid solution (2 drops, 2 molar) was added to a solution of 4-hydrazino-2,3,5,6-tetrafluoro-benzyl alcohol (1.0g) in acetone (10 $cm^3$) and the mixture stirred at the ambient temperature for 30 minutes. The solvent was evaporated under reduced pressure to give a solid which was recrystallised from a hexane/ethyl acetate mixture to give the title compound as a white crystalline solid (1.0g).

Melting point : 127-128°C.

$^1H$ NMR ($CDCl_3$) : 6.86 (1H, broad s); 4.65 (2H, s); 4.4-3.7 (1H, broad); 2.04 (3H, s); 1.98 (3H, s).

$^{19}F$ NMR ($CDCl_3$) : 146.96 to -147.33; -157.35 to -157.74.

EXAMPLE 15

E/Z-4-(ethylidenehydrazino)-2,3,5,6-tetrafluorobenzyl alcohol was prepared from 4-hydrazino-2,3,5,6-tetrafluorobenzyl alcohol and acetaldehyde by a process similar to that described in Example 14.

Melting point : 151°C.

60 MHz $^1H$ NMR ($CDCl_3$) : 7.25 (1H, m); 7.0 (1H, broad); 4.7 (2H, broad s); 2.4 (1H, broad); 1.95 (3H, d).

EXAMPLE 16

This Example illustrates the stages in the preparation of 4-[(1-methylprop-2-yn-1-yl)oxymethyl]-2,3,5,6-tetrafluorobenzyl alcohol.

STAGE 1 : Preparation of 4-(bromomethyl)-2,3,5,6-tetrafluorobenzyl tetrahydropyran-2-yl ether

A mixture of 3-(bromomethyl)-2,3,5,6-tetrafluorobenzyl alcohol (5.4g), dihydropyran (4 $cm^3$) and a catalytic amount of p-toluenesulphonic acid was stirred in dry diethyl ether (20 $cm^3$) at the ambient temperature for 2 hours.

The reaction mixture was poured into water (100 $cm^3$) containing sodium bicarbonate (0.5g) and the mixture was extracted with diethyl ether.

The ethereal extract was washed with water and dried over anhydrous magnesium sulphate.

Removal of magnesium sulphate and evaporation of the solvent under reduced pressure yielded a liquid (7.6g) which slowly crystallised and which was identified as 4-(bromomethyl)-2,3,5,6-tetrafluorobenzyl tetrahydropyran-2-yl ether.

$^1H$ NMR ($CDCL_3$) : 4.83 (2H, t); 4.57 (2H, t); 4.52 (1H, s); 3.90 (1H, m); 3.58 (1H, m); 1.3-1.9 (6H, m).

STAGE 2 : Preparation of (RS)-4-[(1-methylprop-2-yn-1-yl)-oxymethyl]-2,3,5,6-tetrafluorobenzyl tetrahydropyran-2-yl ether

A mixture of 4-(bromomethyl)-2,3,5,6-tetrafluorobenzyl tetrahydropyran-2-yl ether (0.14g), (RS)-3-hydroxybut-1-yne (2 $cm^3$, 55% aqueous solution), sodium hydroxide (1 $cm^3$, 5M aqueous solution), dichloromethane (3 $cm^3$) and a catalytic amount of tetra-n-butylammonium iodide was stirred vigourously at the ambient temperature for 17 hours.

The reaction mixture was diluted with dichloromethane (10 $cm^3$). The layers were separated and the organic layer was passed through phase separation paper to remove residual water. Evaporation of the solvent under reduced pressure yielded an oil (0.12g) identified as (RS)-4-[(1-methylprop-2-yn-1-yl)-oxymethyl]-2,3,5,6-tetrafluorobenzyl tetrahydropyran-2-yl ether.

IR (liquid film) : 3300, 2200 $cm^{-1}$.

STAGE 3 : Preparation of (RS)-4-[(1-methylprop-2-yn-1-yl)-oxymethyl]-2,3,5,6-tetrafluorobenzyl alcohol

A mixture of (RS)-4-[(1-methylprop-2-yn-1-yl)oxymethyl]-2,3,5,6-tetrafluorobenzyl tetrahydropyran-2-yl ether (0.12g), methanol (3 $cm^3$) and hydrochloric acid (2m, 1 $cm^3$) was stirred at the ambient temperature for 2 hours.

The solvent was removed by evaporation and the aqueous residue was extracted with dichloromethane. The organic phase was passed through phase separation paper to remove residual water.

Evaporation of the solvent gave (RS)-4-[(1-methylprop-2-yn-1-yl)oxymethyl]-2,3,5,6-tetrafluorobenzyl alcohol as an oil (0.05g).

IR (liquid film) : 3400, 3300, 2200 $cm^{-1}$.

EXAMPLE 17

4-[(But-2-yn-1-yl)oxymethyl]-2,3,5,6-tetrafluorobenzyl alcohol was prepared by a 3 stage procedure similar to that described in Example 16, using 1-hydroxybut-2-yne in place of (RS)-3-hydroxybut-1-yne.

60 MHz $^1H$ NMR ($CDCl_3$) : 4.84, 4.72 (t + s, 4H), 4.2 (t, 2H) 2.2-2.0 (b, 1H); 1.88 (t, 3H).

Infra red (liquid film) : 3400 $cm^{-1}$ (broad).

EXAMPLE 18

This Example illustrates the stages in the preparation of 4-[(prop-2-yn-1-yl)oxymethyl]-2,3,5,6-tetrafluorobenzyl alcohol.

STAGE 1 : Preparation of methyl 4-[(prop-2- n-1-yl)oxymethyl]-2,3,5,6-tetrafluorobenzoate.

A mixture of methyl 4-bromomethyl-2,3,5,6-tetrafluorobenzoate (0.4g) and 3-hydroxybut-1-yne (0.1g) was stirred vigorously for 2 days in a two phase solvent system which consisted of dichloromethane (3 $cm^3$) and aqueous sodium hydroxide solution (2M, 3 $cm^3$), including a catalytic amount of tetra-n-butylammonium iodide. The mixture was diluted with water and extracted with dichloromethane, and the combined extracts were dried over magnesium sulphate. The solvent was evaporated under reduced pressure to yield an oil which was purified by flash column chromatography (silica), eluting with diethyl ether (1 part) and hexane (10 parts) to give the title product as an oil (0.15g).

Infra red(liquid film) : 3310; 1750 $cm^{-1}$.

STAGE 2 : Preparation of 4-[(prop-2-yn-1-yl)oxymethyl]-2,3,5,6-tetrafluorobenzyl alcohol.

Lithium borohydride (0.03g) was added in portions to a stirred solution of methyl 4-[(prop-2-yn-1-yl)-oxymethyl]-2,3,5,6-tetrafluorobenzoate in dry tetrahydrofuran (3 $cm^3$) under a nitrogen atmosphere until no starting material could be detected by thin layer chromatography. The mixture was then poured into water and was extracted with diethyl ether. The combined extracts were dried over magnesium sulphate. Evaporation of the solvent under reduced pressure yielded the title product as an oil (0.165g).

Infra red (liquid film) : 3400 (broad), 3310 $cm^{-1}$.

EXAMPLE 19

This Example illustrates the preparation of 4-[(prop-2-yn-1-yl)thiomethyl]-2,3,5,6-tetrafluorobenzyl alcohol

Prop-2-yn-1-thiol (0.136g) was slowly added to a stirred mixture of sodium hydride (0.091 g, 56-60% dispersion in oil) in dry N,N-dimethylformamide (2 $cm^3$) under a nitrogen atmosphere at room temperature. After 1 hour the solution was added slowly to a stirred solution of 4-bromomethyl-2,3,5,6-tetrafluorobenzyl alcohol (0.496 g, preparation described in UK Patent Application No 2153819) in dry N,N-dimethylformamide (2 $cm^3$) under a nitrogen atmosphere at room temperature. On completion of the addition the reaction mixture was heated to 60°C for 2 hours. After cooling, the reaction mixture was treated with water (10 $cm^3$) and extracted with diethyl ether (2 x 15 $cm^3$). The combined organic fractions were dried with anhydrous magnesium sulphate and evaporated under reduced pressure to give an orange oil. The oil was fractionated by eluting through a bed of silica gel (Merck 7729) with dichloromethane/ethyl acetate (100:2 by volume) to give the title product as a yellow gum.

GLC Retention Time : 4.62 minutes.

Infra red (liquid film) : 3300, 2955, 1703, 1657, 1484, 1426, 1288, 1229, 1112, 1031, 945, 932, 670, 642 $cm^{-1}$.

EXAMPLE 20

This Example illustrates the stages in an alternative and improved procedure for the preparation of 4-[-(prop-2-yn-1-yl)thiomethyl]-2,3,5,6-tetrafluorobenzyl alcohol.

STAGE 1 : Preparation of 4-hydroxymethyl-2,3,5,6-benzyl mercaptan

A mixture of 4-bromomethyl-2,3,5,6-tetrafluorobenzyl alcohol (0.7g) and thiourea (0.5g) was heated at the reflux temperature with stirring for 2 hours. The reaction mixture was then cooled to the ambient temperature and the resulting solid was collected by filtration, washed with diethyl ether and air dried (1.1g).

The dried solid material was dissolved in methanol, and aqueous sodium hydroxide solution (2M, 10 $cm^3$) was added; the mixture was then heated at the reflux temperature with stirring for 2 hours. The reaction mixture was then cooled to the ambient temperature and stood for 17 hours. The reaction was acidified with aqueous hydrochloric acid (2M) and extracted with diethyl ether, and the combined extracts were dried over magnesium sulphate. Evaporation of the solvent under reduced pressure yielded on oil which crystallised (0.46g).

60 MHz $^1H$ NMR ($CDCl_3$) : 4.8 (s, 2H); 4.6 (bs, 1H); 3.9 (d, 2H); 2.3 (t, 1H).

STAGE 2 : Preparation of 4-[(prop-2-yn-1-yl)thiomethyl]-2,3,5,6-tetrafluorobenzyl alcohol.

A stirred mixture of 4-(hydroxymethyl)-2,3,5,6-tetrafluorobenzyl mercaptan (0.226g), potassium carbonate (0.139g) and propargyl chloride (0.075g) in acetone was heated at the reflux temperature for 15 hours, then cooled to the ambient temperature.

The reaction mixture was poured into water and was extracted with diethyl ether; the combined extracts were dried over magnesium sulphate. Evaporation of the solvent under reduced pressure yielded an oil which was purified by column chromatography (silica), eluting with dichloromethane, to give the title product as a colourless solid (0.21g).

60 MHz $^1H$ NMR ($CDCl_3$) : 4.88 (s, 2H), 4.0 (t, 2H), 3.32 (d, 2H); 2.5 (bs, 1H), 2.28 (t, 1H).

## EXAMPLE 21

This Example illustrates the preparation of 4-[(prop-2-ylideneamino)oxymethyl]-2,3,5,6-tetrafluorobenzyl alcohol.

4-Bromomethyl-2,3,5,6-tetrafluorobenzyl alcohol (1.002 g, preparation described in UK Patent Application 2153819 A), acetone oxime (0.311 g), potassium t-butoxide (0.424 g) and dry dimethoxyethane (3 $cm^3$) were heated at the reflux temperature for 2 hours. The reaction was diluted with dichloromethane and water, the organic layer separated and the aqueous layer was extracted with further dichloromethane. The combined organic extracts were washed with water, dried over anhydrous magnesium sulphate and the solvent evaporated under reduced pressure to give the title product as an orange oil (0.835 g).

GLC Retention Time : 3.62 minutes

Infra Red (thin film) : 3376, 2957, 1734, 1487, 1369, 1286, 1032, 892, 828 $cm^{-1}$

## EXAMPLE 22

This Example illustrates the stages in the preparation of 4-(2-cyanoethyl)-2,3,5,6-tetrafluorobenzyl alcohol.

STAGE 1 : Preparation of 2-[4-(2-hydroxyethyl)-2,3,5,6-tetrafluorobenzyloxy]tetrahydropyran.

A solution of n-butyllithium (2.5M solution in n-hexane, 4.0 $cm^3$) was added dropwise over 30 minutes to a stirred solution of 2-(4-bromo-2,3,5,6-tetrafluorobenzyloxy)tetrahydropyran (3.43g, prepared from 4-bromo-2,3,5,6-tetrafluorobenzyl alcohol according to the method described in Stage 1 of Example 16) in dry tetrahydrofuran (5 $cm^3$); the temperature of the mixture was maintained at -70 °C under a nitrogen atmosphere during the addition. Ethylene oxide was then passed through the reaction mixture for two minutes (exothermic reaction). The mixture was then allowed to warm to ambient temperature over 30 minutes. The reaction mixture was poured into water, extracted with diethyl ether, and the combined ethereal extracts were dried over magnesium sulphate. The solvent was removed by evaporation under

reduced pressure to give the title intermediate as a yellow oil (2.63g).

$^1$H NMR ($CDCl_3$) : 4.88-4.78 (m, 2H); 4.60 (d, 1H); 3.96-3.84 (m, t, 3H); 3.56 (m, 1H); 3.02 (t, 2H); 1.9-1.48 (m, 6H).

STAGE 2 : Preparation of 2-[4-(2-bromoethyl)-2,3,5,6-tetra-fluorobenzyloxy]tetrahydropyran.

A solution of 1,2-dibromo-1,1,2,2-tetrachloroethane (0.58g) was added to a stirred mixture of triphenylphosphine (0.47g) and 2-[4-(2-hydroxyethyl)-2,3,5,6-tetrafluorobenzyloxy]tetrahydropyran (0.5g) in dry diethyl ether and the reaction mixture was maintained at a temperature of 0°C under a nitrogen atmosphere for 10 to 15 minutes. The reaction mixture was then filtered, and the filtrate was evaporated under reduced pressure. The crude, oily product was purified by column chromatography, eluting with dichloromethane. The component having an Rf of 0.52 by TLC on silica gel, eluting with dichloromethane was characterised as the title product, and was used directly in Stage 3.

STAGE 3 : Preparation of 2-[4-(2-cyanoethyl)-2,3,5,6-tetrafluorobenzyloxy]tetrahydropyran.

A mixture of 2-[4-(2-bromoethyl)-2,3,5,6-tetrafluorobenzyloxy]tetrahydropyran (0.5g), sodium cyanide (0.1g) and dimethyl sulphoxide was stirred at the ambient temperature for 17 hours. The reaction mixture was poured into water and extracted with diethyl ether. The combined extracts were dried over magnesium sulphate and the solvent was evaporated under reduced pressure to yield an oil (0.35g) which was used without further purification in Stage 4.

Infra red (liquid film) : 2200 $cm^{-1}$.

STAGE 4 : Preparation of 4-(2-cyanoethyl)-2,3,5,6-tetrafluorobenzyl alcohol.

A mixture of 2-[4-(2-cyanoethyl)-2,3,5,6-tetrafluorobenzyloxy]tetrahydropyran (0.35g), aqueous hydrochloric acid (2M, 1 $cm^3$), and methanol (5 $cm^3$) was stirred at the ambient temperature for 15 minutes. The reaction mixture was diluted with water and was extracted with diethyl ether. The combined extracts were dried over magnesium sulphate. The solvent was removed by evaporation under reduced pressure to give an oil (0.28g) which was purified by flash chromatography using silica and eluting with dichloromethane. The component having an Rf of 0.13 by TLC on silica gel, eluting with dichloromethane, was characterised as the title product (0.17g) and was isolated as an oil which crystallised on standing.

$^1$H NMR ($CDCl_3$) : 4.82 (d, 2H); 3.13 (t, 2H); 2.69 (t, 2H); 2.08 (t, 1H).

EXAMPLE 23

This Example illustrates the preparation of 4-[N-(prop-2-yn-1-yl)-N-methyl]aminomethyl-2,3,5,6-tetrafluorobenzyl alcohol.

A mixture of 4-(bromomethyl)-2,3,5,6-tetrafluorobenzyl alcohol (1.0g) and N-(prop-2-yn-1-yl)-N-methylamine (0.96g) was dissolved in acetonitrile and stirred at the ambient temperature for 1 hour.

The solvent was evaporated under reduced pressure to yield a brown solid which was dissolved in water and neutralised with saturated aqueous sodium bicarbonate solution. This aqueous mixture was then extracted with chloroform and the extracts dried over magnesium sulphate. The solvent was evaporated under reduced pressure to yield the title product as an oil (0.87g).

GLC retention timees :

a) 4-)bromomethyl)-2,3,5,6-tetrafluorobenzyl alcohol: 2.23 minutes.

b) product : 3.43 minutes.

## EXAMPLE 24

This Example illustrates the preparation of 4-(acetylthiomethyl)-2,3,5,6-tetrafluorobenzyl alcohol.

A mixture of potassium carbonate (0.5g), thioacetic acid (0.31g) and 4-(bromomethyl)-2,3,5,6-tetrafluorobenzyl alcohol was stirred at the ambient temperature for 1.5 hours. The reaction mixture was poured into water and extracted with diethyl ether. The combined extracts were washed with saturated sodium carbonate and dried over anhydrous magnesium sulphate. Evaporation of the solvent under reduced pressure gave an oil which was purified by flash column chromatography (silica) eluting with a 1:1 by volume mixture of diethyl ether and hexane to give the title product (0.68g) as a pale yellow crystalline solid.

60 MHz $^1$H NMR ($CDCl_3$) : 4.9-4.76 (d, 2H); 4.24 (t, 2H); 2.4 (s, 3H); 2.04 (t, 1H).

## EXAMPLE 25

The following method was used to prepare the esters described below from the appropriate acid and alcohol.

Equimolar amounts of the acid and the alcohol, and a catalytic quantity of N,N-dimethylaminopyridine were mixed in dichloromethane and an equimolar amount of dicyclohexyl-carbodiimide was added to the stirred mixture. Progress of the reaction was monitored by thin layer chromatography. On completion of the reaction, the solid precipitate was removed by filtration and the filtrate concentrated by evaporation under reduced pressure. The crude reaction prouct was purified by column chromatography on a silica gel support, usually eluting with dichloromethane.

The following esters were obtained by the general method described above.

(i) 4-(Formyloxymethyl)-2,3,5,6-tetrafluoromethyl (1RS, cis)-3-(Z-2-chloro-3,3,3-trifluoroprop-1-en-1-yl)-2,2-dimethylcyclopropanecarboxylate, (Product VIII)

$^1$H NMR ($CDCl_3$) : 8.09 (1H, s); 6.89 (1H, d) 5.33 2H, s): 5.25 (2H, q): 2.12 (1H, t): 1.98 (1H, d): 1.30 (6H, s}.

(ii) 4-(Formyloxymethyl)-2,3,5,6-tetrafluoromethyl (1RS, trans)-3-(Z-2-chloro-3,3,3-trifluoroprop-1-en-1-yl)-2,2-dimethylcyclopropanecarboxylate, (Product IX)

$^1$H NMR ($CDCl_3$) : 8.09 (1H, s); 6.14 (1H, d); 5.33 (2H, s; 5.26 (2H, s); 2.45 (1H, q); 1.78 (1H, d); 1.34 (3H, s); 1.24 (3H, s).

(iii) 4-(Azidomethyl)-2,3,5,6-tetrafluorobenzyl (1RS, cis)-3-(Z-2-chloro-3,3,3-trifluoroprop-1-en-1-yl)-2,2-dimethylcyclopropanecarboxylate, (Product X)

90 MHz $^1$H NMR ($CDCl_3$) : 6.86 (1H, d); 5.23 (2H, s); 4.48 (2H, s); 1.9-2.3 (2H, m); 1.30 (6H, s) .
Infra red (liquid film) : 2100, 1730 $cm^{-1}$.

(iv) 4-(Azidomethyl)-2,3,5,6-tetrafluorobenzyl (1RS, trans)-3-(Z-2-chloro-3,3,3-trifluoroprop-1-en-1-yl)-2,2-dimethylcyclopropanecarboxylate, (Product XI)

$^1H$ NMR ($CDCl_3$) : 6.12 (1H, d); 5.28 (2H, s); 4.49 (2H, s); 2.44 (1H, q); 1.78 (1H, d); 1.34 (3H, s); 1.24 (3H, s).

Infra red (liquid film) : 2100, 1730 $cm^{-1}$.

(v) 4-(Cyanomethyl)-2,3,5,6-tetrafluorobenzyl (1RS, cis)-3-(Z-2-chloro-3,3,3-trifluoroprop-1-en-1-yl)-2,2-dimethylcyclopropanecarboxylate, (Product XII)

90 MHz $^1H$ NMR ($CDCl_3$) : 6.86 (1H, d); 5.25 (2H, s); 3.83 (2H, s); 1.9-2.3 (2H, m); 1.30 (6H, s).

Infra red (liquid film) : 2200, 1730 $cm^{-1}$.

(vi) 4-(prop-2-ylidenehydrazino)-2,3,5,6-tetrafluorobenzyl (1RS, cis)-3-(Z-2-chloro-3,3,3-trifluoroprop-1-en-1-yl)-2,2-dimethylcyclopropanecarboxylate (Product XIII).

$^1H$ NMR ($CDCl_3$) : 6.95 (1H, d); 6.73 (1H, broad); 5.2 (2H, s); 1.6-2.4 (8H, m); 1.30 (6H, s).

Infra red (liquid film) : 3300, 1730, 1660 $cm^{-1}$.

(vii) E/Z-4-(ethylidenehydrazino)-2,3,5,6-tetrafluorobenzyl (1RS, cis)-3-(Z-2-chloro-3,3,3-trifluoroprop-1-en-1-yl)-2,2-dimethylcyclopropanecarboxylate (Product XIV).

60 MHz $^1H$ NMR ($CDCl_3$) : 7.2 (2H, broad); 6.9 (1H, d); 5.2 (2H, s); 1.6-2.3 (5H, m); 1.30 (6H, s).

Infra red (liquid film) : 3300, 1730, 1660 $cm^{-1}$.

(viii)4-(prop-2-ylidenehydrazino)-2,3,5,6-tetrafluorobenzyl (1RS, cis)-3-(2,2-dichloroethenyl)-2,2-dimethylcyclopropanecarboxylate (Product XV).

Infra red (liquid film) : 3300, 1730, 1660 $cm^{-1}$.

(ix) 4-Ethynyl-2,3,5,6-tetrafluorobenzyl (1RS, cis)-3-(Z-2-chloro-3,3,3-trifluoroprop-1-en-1-yl)-2,2-dimethylcyclopropanecarboxylate (Product XVI).

90 MHz $^1H$ NMR ($CDCl_3$) : 6.86 (1H, d); 5.22 (2H, s); 3.68 (1H, s); 1.9-2.3 (2H, m); 1.30 (6H, s).

$^{19}F$ NMR ($CDCl_3$) : -136.7 (q); -142.9 (q).

Infra red (liquid film) : 3320, 1730 $cm^{-1}$.

(x) 4-Ethynyl-2,3,5,6-tetrafluorobenzyl-2,2,3,3-tetramethylcyclopropanecarboxylate, (Product XVII)

$^1$H NMR ($CDCl_3$) : 5.20 (2H, s); 3.65 (1H, s); 1.55 (1H, s); 1.24 (12H, d).

$^{19}$F NMR ($CDCl_3$) : -137.1 (q); -142.9 (q).

Infra red (liquid film) : 3300, 1730 $cm^{-1}$.

(xi) (Rs)-4-[(l-methylprop-2-yn-1-yl)oxymethyl]-2,3,5,6-tetrafluorobenzyl (1RS,cis)-3-(Z-2-chloro-3,3,3-trifluoroprop-1-en-1-yl)-2,2-dimethylcyclopropanecarboxylate, (Product XVIII).

$^1$H NMR ($CDCl_3$) : 6.90 (1H, d); 5.25 (2H, q) 4.90, 4.64 (2H, 2d); 4.30 (1H, q); 2.51 (1H, s); 2.20 (1H, t); 1.98 (1H, d); 1.47 (3H, d); 1.30 (6H, s).

(xii) 4-[(But-2-yn-1-yl)oxymethyl]-2,3,5,6-tetrafluorobenzyl (1RS,cis)-3-(Z-2-chloro-3,3,3-trifluoroprop-1-en-1-yl)-2,2-dimethylcyclopropanecarboxylate, (Product XIX).

$^1$H NMR ($CDCl_3$) : 6.90 (1H, d); 5.25 (2H, q); 4.72 (2H, s); 4.20 (2H, s); 2.20 (1H, t); 1.98 (1H, d); 1.88 (3H, s): 1.29 (3H, s).

(xiii) 4-[(Prop-2-yn-1-yl)oxymethyl]-2,3,5,6-tetrafluorobenzyl (1RS,cis)-3-(Z-2-chloro-3,3,3-trifluoroprop-1-en-1-yl)-2,2-dimethylcyclopropanecarboxylate, (Product XX).

$^1$H NMR ($CDCl_3$) : 6.87 (1H, d); 5.22 (1H, q); 4.72 (2H, s); 4.21 (2H, d); 2.50 (1H, t); 2.17 (1H, t); 1.94 (1H, d); 1.30 (3H, s).

(xiv) 4-(2-cyanoethyl)-2,3,5,6-tetrafluorobenzyl (1RS, cis)-3-(Z-2-chloro-3,3,3-trifluoroprop-1-en-1-yl)-2,2-dimethylcyclopropanecarboxylate (Product XXI).

$^1$H NMR ($CDCl_3$) : 6.8 (d, 1H); 5.24 (q, 2H); 3.12 (t, 2H); 2.7 (t, 2H); 2.18 (t, 1H); 1.95 (d, 1H); 1.30 (s, 6H).

(xv) 4-[(prop-2-ylideneamino)oxymethyl]-2,3,5,6-tetrafluorobenzyl (1RS, cis)-3-(Z-2-chloro-3,3,3-trifluoroprop-1-en-1-yl-2,2-dimethylcyclopropanecarboxylate (Product XXII).

90 MHz $^1$H NMR ($CDCl_3$) : 6.84 (d, 1H) 5.2 (s, 2H); 5.12 (s, 2H); 2.2-1.9 (m, 2H); 1.84 (d, 6H); 1.32 (s, 6H).

(xvi) 4-[(prop-2-ylidenamino)oxymethyl]-2,3,5,6-tetrafluorobenzyl (1RS, trans)-3-(Z-2-chloro-3,3,3-trifluoroprop-1-en-1-yl)-2,2-dimethylcyclopropanecarboxylate (Product XXIII).

90 MHz $^1$H NMR ($CDCl_3$) : 6.12 (d, 1H); 5.24 (s, 2H); 5.12 (s, 2H); 2.5-2.28 (m, 1H); 1.84 (d, 6H; 1.32 (s, 3H); 1.2 (s, 3H).

(xvii) 4-[(N-(prop-2-yn-1-yl)-N-methyl)aminomethyl]-2,3,5-6-tetrafluorobenzyl (1RS, cis)-3-(Z-2-chloro-3,3,3-trifluoroprop-1-en-1-yl)-2,2-dimethylcyclopropanecarboxylate (Product XXIV).

$^1$H NMR ($CDCl_3$) : 6.9 (d, 1H); 5.24 (q, 2H); 3.76 (s, 2H); 3.4 (d, 2H); 2.37 (s, 3H); 2.30 (t, 1H); 2.2 (t, 1H); 1.95 (d, 1H); 1.29 (s, 6H).

(xviii) 4-[(prop-2-yn-1-yl)thiomethyl]-2,3,5,6-tetra fluorobenzyl (1RS, cis)-3-(Z-2-chloro-3,3,3-trifluoroprop-1-en-1-yl)-2,2-dimethylcyclopropanecarboxylate (Product XXV).

400 MHz $^1$H NMR ($CDCl_3$) : 6.9 (d, 1H); 5.25 (q, 2H); 3.95 (s, 2H); 3.28 (d, 2H); 2.27 (t, 1H); 2.2 (t, 1H); 1.95 (d, 1H); 1.3 (s, 6H).

(xix) 4-(acetylthiomethyl)-2,3,5,6-tetrafluorobenzyl (1RS, cis)-3-(Z-2-chloro-3,3,3-trifluoroprop-1-en-1-yl)-2,2-dimethylcyclopropanecarboxylate (Product XXVI).

$^1$H NMR ($CDCl_3$) : 6.9 (d, 1H); 5.24 (q, 2H); 4.24 (s, 2H); 2.37 (s, 2H); 2.2 (t, 1H); 1.95 (d, 1H); 1.29 (s, 6H).

(xx) 4-(acetylthiomethyl)-2,3,5,6-tetrafluorobenzyl (1RS, trans)-3-(Z-2-chloro-3,3,3-trifluoroprop-1-en-1-yl)-2,2-dimethylcyclopropanecarboxylate (Product XXVII).

$^1$H NMR ($CDCl_3$); 6.12 (d, 1H); 5.24 (s, 2H); 4.24 (s, 2H; 2.44 (m, 1H); 2.37 (s, 1H); 1.76 (d, 1H); 1.36 (s, 3H); 1.23 (s, 3H).

(xxi) 4-(azidomethyl)-2,3,5,6-tetrafluorobenzyl (1RS, cis/trans)-3-(Z-2,3,3,3-tetrafluoroprop-1-en-1-yl)2,2-dimethylcyclopropanecarboxylate; cis:trans ratio 1:9 (Product XXVIII).

400 MHz $^1$H NMR ($CDCl_3$) : 5.49-5.40 (double d, 0.1H); 5.34-5.20 (m, 2.9H); 4.50 (s, 2H); 2.40-2.32 (double d, 0.9H); 2.18 (t, 0.1H); 1.92 (d, 0.1H); 1.7 (d, 0.9H); 1.27 (d, 2.7H); 1.22 (d, 0.3H).

## EXAMPLE 26

This Example illustrates the insecticidal properties of the Products of this invention.

The activity of the Product was determined using a variety of insect pests. The Product was used in the form of liquid preparations containing 500, 250 or 100 parts per million (ppm) by weight of the Product. The preparations were made by dissolving the Product in acetone and diluting the solutions with water containing 0.01% by weight of a wetting agent sold under the trade name "LISSAPOL" NX until the liquid preparations contained the required concentration of the Product. "Lissapol" is a Registered Trade Mark.

The test procedure adopted with regard to each pest was basically the same and comprised supporting a number of the pests on a medium which was usually a host plant or a foodstuff on which the pests feed, and treating either or both the pests and the medium with the preparations. The mortality of the pests was then assessed at periods usually varying from one to three days after the treatment.

The results of the tests are given in Table III for each of the Products, at the rate in parts per million given in the second column as a grading of mortality designated as A, B or C wherein A indicates 80-100% mortality, B indicates 50-79% mortality and C indicates less than 50% mortality.

In Table 111 the pest organism used is designated by a letter code and the pests species, the support medium or food, and the type and duration of test is given in Table II.

TABLE II

| CODE LETTERS (Table IV) | TEST SPECIES | SUPPORT MEDIUM/FOOD | TYPE OF TEST | DURATION (days) |
|---|---|---|---|---|
| TUa | Tetranychus urticae (spider mites - adult) | French bean leaf | Contact | 3 |
| MP | Myzus persicae (aphids) | Chinese Cabbage leaf | Contact | 3 |
| NC | Nephotettix cincticeps (green leaf hopper - nymphs) | Rice plant | Contact | 3 |
| HV | Heliothis virescens (tobacco budworm - larvae) | Cotton leaf | Residual | 3 |
| DB | Diabrotica balteata (rootworm larvae) | Filter paper/maize seed | Residual | 3 |
| BG | Blattella germanica (cockroach nymphs) | Plastic pot | Residual | 3 |
| MD | Musca domestica (houseflies - adults) | Cotton wool/sugar | Contact | 1 |
| SP | Spodoptera exigua (lesser army worm - larvae) | Cotton leaf | Residual | 3 |

"Contact" test indicates that both pests and medium were treated and "residual" indicates that the medium was treated before infestation with the pests.

TABLE III

| PRODUCT | RATE (ppm) | Tua | MP | NC | HV | DB | BG | MD | SP |
|---|---|---|---|---|---|---|---|---|---|
| I | 100 | C | - | - | B | A | - | C | - |
| II | 100 | C | A | A* | A | A | A | A | A |
| III | 100 | A | A | A | A | A | A | C | A |
| IV | 100 | A | A | A | A | C | A | C | A |
| V | 100 | A | A | A | A | A | A | A | A |
| VI | 100 | B | A | A | A | A | A | A | A |
| VII | 100 | A | A | A | A | A | A | C | A |
| VIII | 100 | A | A | A | A | A | A | A | A |
| IX | 100 | C | A | A+ | A+ | C+ | A | C** | A+ |
| X | 100 | A | A | A | A | A | A | A | A |
| XII | 100 | A | C | A | A | A | A | A | A |
| XIII | 100 | C | B | A* | A | A | A | A | - |
| XIV | 500 | C | A | - | A | A | A | A | - |
| XV | 500 | - | A | A* | A | A | A | A | - |
| XVI | 100 | B | A | A* | A | A | A | A | A |

TABLE III (Cont/d)

| PRODUCT | RATE (ppm) | Tua | MP | NC | HV | DB | BG | MD | SP |
|---|---|---|---|---|---|---|---|---|---|
| XVII | 500 | A | C | A* | A | C | A | A | A |
| XVIII | 100 | C | A | A | A | A | A | A | A |
| XIX | 100 | B | A | A | A | A | A | B | A |
| XX | 100 | A | A | A | A | A | A | A | A |
| XXI | 100 | C | A | A+ | A+ | C+ | C | C** | C+ |
| XXII | 100 | A | A | A+ | A+ | A+ | C | A** | A+ |
| XXIII | 100 | A | A | A+ | C+ | A+ | C | C** | A+ |
| XXIV | 100 | B | A | A+ | A+ | A+ | B | A** | A+ |
| XXV | 100 | A | A | A+ | A+ | A+ | A | A** | A+ |
| XXVI | 100 | A | A | A+ | A+ | A+ | A | A** | A+ |
| XXVII | 100 | A | A | A+ | A+ | A+ | C | A** | A+ |
| XXVIII | 100 | C | A | A+ | A+ | A+ | A | A** | A+ |

* Nephotettix cincticeps replaced by Nilaparvata lugens in this test

\+ Test duration of 2 days

** Test duration of 3 days

## EXAMPLE 27

This Example illustrates the knockdown activity of the compounds according to the invention.

<u>Blattella germanica</u> knockdown test :

The test compound was dissolved in acetone (2 $cm^3$) and the solution diluted to a concentration of 250 ppm with kerosene. 1 $cm^3$ Of this preparation was sprayed onto 10 <u>Blattella germanica</u> (adult males) held in a netted plastic pot in a Burkhard potter Tower. Assessment of knockdown was performed at intervals of 5 minutes up to a total of 20 minutes. On removal from the Burkhard Potter Tower, the insects were held at 25°C and 65% relative humidity for 48 hours, and an assessment of mortality performed. Each test was repeated. Results are given in Table IV.

In some tests, additional knockdown assessment was performed at intervals of 1, 2, 3 and 4 minutes, and $KT_{50}$ and $KT_{90}$ values determined (the time taken, in minutes, to knockdown 50% and 90% respectively of test insects).

TABLE IV

| TEST COMPOUND | TEST | % KNOCKDOWN OBSERVED | | | | % MORTALITY | $KT_{50}$ | $KT_{90}$ |
|---|---|---|---|---|---|---|---|---|
| | | 5 min | 10 min | 15 min | 20 min | 48 hrs | mins | mins |
| Product II | 1 | 0 | 60 | 90 | 90 | 20 | | |
| | 2 | 40 | 60 | 100 | | 100 | | |
| Product III | 1 | 100 | | | | 100 | | |
| | 2 | 100 | | | | 100 | | |
| Product VI | 1 | 100 | | | | 100 | | |
| | 2 | 30 | 30 | 70 | 100 | 100 | | |
| Product IX | 1 | 100 | | | | 100 | | |
| | 2 | 50 | 100 | | | 100 | | |
| Product X | 1 | 30 | 70 | 100 | | 100 | | |
| | 2 | 70 | 80 | 100 | | 100 | | |
| Product XII | 1 | 100 | | | | 100 | | |
| | 2 | 30 | 100 | | | 100 | | |
| Product XIV | 1 | 0 | 50 | 100 | | 50 | | |
| | 2 | 20 | 100 | | | 90 | | |
| Product XVI | 1 | 100 | | | | 100 | | |
| | 2 | 50 | 100 | | | 100 | | |
| Product XXI | 1 | 60 | 100 | | | 60 | 4.2 | 8.8 |
| | 2 | 70 | 100 | | | 100 | 3.6 | 8.2 |

TABLE IV (Cont/d)

| TEST COMPOUND | TEST | % KNOCKDOWN OBSERVED | | | | % MORTALITY | $KT_{50}$ | $KT_{90}$ |
|---|---|---|---|---|---|---|---|---|
| | | 5 min | 10 min | 15 min | 20 min | 48 hrs | mins | mins |
| Product XXII | 1 | 70 | 100 | | | 100 | 3.6 | 8.3 |
| | 2 | 100 | | | | 100 | 2.5 | 4.5 |
| Product XXIII | 1 | 10 | 10 | 10 | 10 | 100 | >20 | - |
| | 2 | 30 | 100 | | | 100 | 6.4 | 9.3 |
| Product XXIV | 1 | 60 | 100 | | | 100 | 4.2 | 8.8 |
| | 2 | 50 | 100 | | | 70 | 5.0 | 9.0 |
| Product XXV | 1 | 100 | | | | 100 | 2.5 | 4.5 |
| | 2 | 70 | 100 | | | 40 | 3.6 | 8.3 |
| Product XXVI | 1 | 60 | 100 | | | 100 | 4.2 | 8.8 |
| | 2 | 0 | 40 | 60 | 100 | 100 | 12.4 | 18.7 |
| Product XXVII | 1 | 10 | 100 | | | 100 | 7.2 | 9.4 |
| | 2 | 0 | 20 | 40 | 40 | 80 | >20 | >20 |
| Product XXVIII | 1 | 30 | 100 | | | 100 | 6.4 | 9.3 |
| | 2 | 0 | 30 | 70 | 100 | 100 | 12.6 | 18.3 |
| Tetramethrin | | 80* | 100* | | | 50 | 1.7** | 3.2** |

* Meaned values from 27 obsrvations.

** Meaned values from 3 assessments.

<u>Musca domestica</u> knockdown test :

1 $cm^3$ Of a 30 ppm solution of the test chemical in acetone was sprayed into a Kearns and March chamber containing 20 <u>Musca domestica</u> (adult females). Knockdown was observed over a period of 10 minutes and $KT_{50}$ and $KT_{90}$ values (the time taken for 50% and 90% of the insects to be knocked down) were calculated from the observations. The results given in Table V represent average KT values of 2 replicates except where otherwise stated.

TABLE V

| TEST COMPOUND | $KT_{50}$ (mins) | $KT_{90}$ (mins) |
|---|---|---|
| Product II | 2.7 | 5.0 |
| Product III | 10 | 10 |
| Product VI | 7.0 | 7.7* |
| Product IX | 10* | 10* |
| Product X | 5.0* | 9.0* |
| Product XII | 6.8 | 9.8 |
| Product XIV | 6.6 | 9.4 |
| Product XVI | 4.6* | 7.0* |
| Product XVII | 4.1 | 7.5 |
| Tetramethrin | 4.0** | 7.5** |

* Indicates result of single replicate
** Indicates average of results of 7 tests

## Claims

1. A compound of formula (I) :

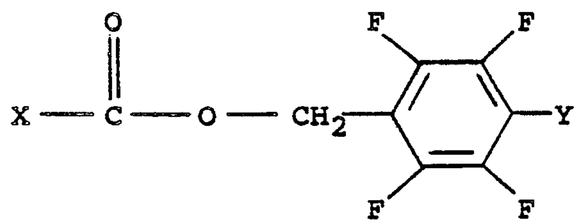

(I)

or a stereoisomer thereof, wherein X represents a group of formula :

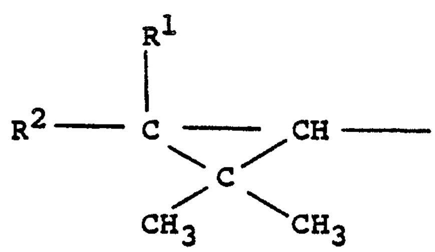

where either (i) $R^1$ and $R^2$ are each selected from hydrogen, halo and $C_{1-4}$ alkyl or (ii) $R^1$ is hydrogen and $R^2$ represents either a group of formula :

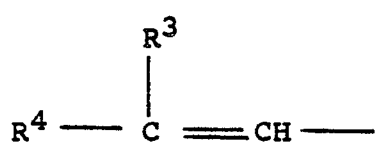

or a group of formula :

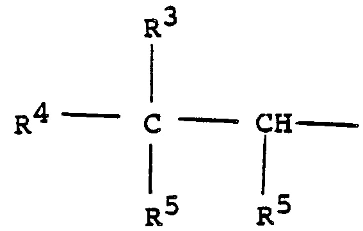

where $R^3$ and $R^4$ are each selected from hydrogen, methyl, halo and $C_{1-2}$ haloalkyl containing at least two fluorine atoms, and $R^5$ is selected from chloro and bromo; and either

(i) Y represents a group of formula $-CH_2-Z$ wherein Z is selected from halo, cyano, azido and cyanomethyl, or Z is selected from a group of formula $-OCOR^6$, a group of formula $-SCOR^7$, a group of formula $-OCO_2R^7$, a group of formula $-OR^8$, a group of formula $-SR^8$, a group of formula $-N(R^{15})R^8$, a group of formula $-OSO_2R^9$, and a group of formula $-ON=C(R^{13})R^{14}$, wherein $R^6$ represents hydrogen, $C_{1-4}$ alkyl or $C_{1-4}$ haloalkyl, $R^8$ represents propargyl optionally substituted with one or more methyl groups, $R^7$ and $R^9$ represent $C_{1-4}$ alkyl or $C_{1-4}$ haloalkyl, and $R^{13}$ $R^{14}$ and $R^{15}$ represent $C_{1-4}$ alkyl, or

(ii) Y represents a group of formula $-C{\equiv}CR^{10}$ or a group of formula $-NH-N=C(R^{11})R^{12}$ wherein $R^{10}$, $R^{11}$ and $R^{12}$ are selected from hydrogen, $C_{1-4}$ alkyl and $C_{1-4}$ haloalkyl.

2. A compound according to claim wherein X represents the group of formula :

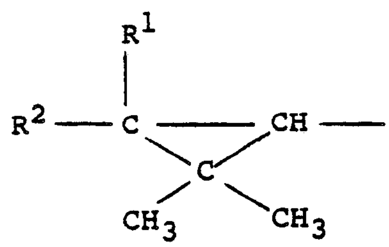

in which either (i) $R^1$ and $R^2$ both represent methyl, or (ii) $R^1$ represents hydrogen and $R^2$ represents the group of formula $R^4(R^3)C=CH-$ in which $R^3$ and $R^4$ are each selected from hydrogen, methyl, halo and trifluoromethyl.

3. A compound according to claim 1 or claim 2 wherein the group Y has a value selected from fluoromethyl, chloromethyl, bromomethyl, cyanomethyl, azidomethyl, 2-cyanoethyl, formyloxymethyl, acetoxymethyl, acetylthiomethyl, trifluoroacetoxymethyl, methoxycarbonyloxymethyl, (l-methylprop-2-yn-1-yl)-oxymethyl, (but-2-yn-1-yl)oxymethyl, (prop-2-yn-1-yl)oxymethyl, (prop-2-yn-1-yl)thiomethyl, [N-(prop-2-yn-1-yl)-N-methyl]aminomethyl, methanesulphonyloxymethyl, (prop-2-ylideneamino)oxymethyl, ethynyl, prop-2-ylidenhydrazino and ethylidenehydrazino.

4. A compound of formula (V) :

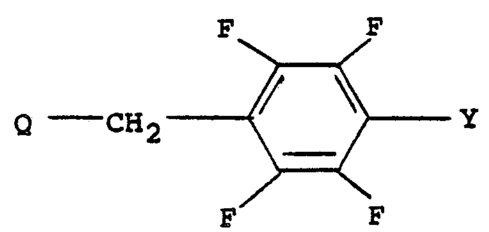

(V)

wherein Q represents hydroxy or halo and either :

(i) Y represents a group of formula $-CH_2-Z$ wherein Z is selected from fluoro, cyano, azido and cyanomethyl, or Z is selected from a group of formula $-OCOR^6$, a group of formula $-SCOR^7$, a group of formula $-OCO_2R^7$, a group of formula $-OR^8$, a group of formula $-SR^8$, a group of formula $-N(R^{15})R^8$, a group of formula $-OSO_2R^9$, and a group of formula $-ON=C(R^{13})R^{14}$, wherein $R^6$ represents hydrogen, $C_{1-4}$ alkyl or $C_{1-4}$ haloalkyl, $R^8$ represents propargyl optionally substituted with one or more methyl groups, $R^7$ and $R^9$ represent $C_{1-4}$ alkyl or $C_{1-4}$ haloalkyl, and $R^{13}$, $R^{14}$ and $R^{15}$ represent $C_{1-4}$ alkyl, or

(ii) Y represents a group of formula -C≡$R^{10}$ or a group of formula -NH-N=C($R^{11}$)$R^{12}$ wherein $R^{10}$, $R^{11}$ and $R^{12}$ are selected from hydrogen, $C_{1-4}$ alkyl and $C_{1-4}$ haloalkyl.

5. A compound according to claim 4 wherein the group Y has a value selected from fluoromethyl, cyanomethyl, azidomethyl, 2-cyanoethyl, formyloxymethyl, acetoxymethyl, acetylthiomethyl, trifluoroacetoxymethyl, methoxycarbonyloxymethyl, (1-methylprop-2-yn-1-yl)oxymethyl, (but-2-yn-1-yl)oxy-methyl, (prop-2-yn-1-yl)oxymethyl, (prop-2-yn-1-yl)-thiomethyl, [N-(prop-2-yn-1-yl)-N-methyl]aminomethyl, methanesulphonyloxymethyl, (prop-2-ylideneamino)oxymethyl, ethynyl, prop-2-ylidenehydrazino and ethylidenehydrazino.

6. A process for preparing a compound according to claim 1 wherein either

(a) an acid of formula X-COOH, wherein X has any of the meanings given in claim 1, is reacted with an alcohol of formula (III) :

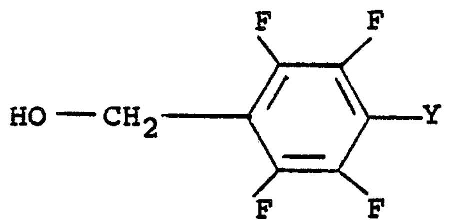

(III)

wherein Y has any of the meanings given in claim 1, the reaction taking place in the presence of an acid catalyst or a dehydrating agent; or

(b) an acid halide of formula X-COHal wherein X has any of the meanings given in claim 1 and Hal represents a halogen atom is reacted with an alcohol of formula (III) wherein Y has any of the meanings given in claim 1, the reaction taking place in the presence of a base; or

(c) an acid of formula X-COOH, wherein X has any of the meanings given in claim 1 is reacted with either (i) a compound of formula (IV) :

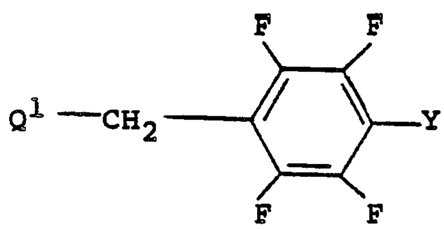

(IV)

wherein $Q^1$ represents a halogen atom and Y has any of the meanings given in claim 1, or with a quaternary ammonium salt derived from such halides by reaction with a tertiary amine, or (ii) a compound of formula (IV) wherein $Q^1$ represents the mesylate or tosylate group.

(d) a lower alkyl ester of formula X-COOQ where X has any of the meanings given in claim 1 and Q represents a lower alkyl group containing up to six carbon atoms, is heated with an alcohol of formula (III) to effect a transesterification reaction.

7. An insecticidal composition comprising an insecticidally effective amount of a compound according to claim 1 in association with an insecticidally inert diluent or carrier.

8. A method of combating insect pests at a locus which comprises applying to the locus an insecticidally effective amount of a composition according to claim 7.